# EUROPEAN PATENT SPECIFICATION

(11) **EP 1 607 081 B1**
(45) Date of publication and mention of the grant of the patent: **22.02.2012**
(21) Application number: 04721715.3
(22) Date of filing: 18.03.2004
(51) Int. Cl.: A61K 8/58, A61Q 1/00, A61Q 1/06, A61Q 1/10, A61Q 1/14, A61Q 17/04

(54) **COSMETIC PREPARATION**
KOSMETISCHE ZUBEREITUNG
PREPARATION COSMETIQUE

(30) Priority: 19.03.2003 JP 2003074978; 28.04.2003 JP 2003123263; 28.04.2003 JP 2003123265; 28.04.2003 JP 2003123264; 28.04.2003 JP 2003123262; 28.04.2003 JP 2003123266; 23.06.2003 JP 2003177608
(43) Date of publication of application: 21.12.2005
(73) Proprietor: Kao Corporation, Chuo-ku Tokyo 103-8210 (JP); Shin-Etsu Chemical Co., Ltd., Chiyoda-ku, Tokyo 100-0004 (JP)
(72) Inventor: KURODA, Akihiro, Yokohama-shi, Kanagawa; 226-0011 (JP); SAKUTA, Koji, Silicone-Electronic Mat. Resear. Ctr, Matsuida-m., Usui-gun, Gunma 379-0222 (JP)
(74) Representative: Jansen, Cornelis Marinus
(86) International application number: PCT/JP2004/003623
(87) International publication number: WO 2004/082644

(56) References cited:
- EP-A- 0 915 123
- WO-A1-01/15658
- US-A- 2 567 110

## Description

### FIELD OF THE INVENTION

The present invention relates to a process for making a cosmetic, comprising tetraquistrimethylsiloxysilane. The cosmetic does not give dry feel or irritation to the skin. It is comfortable to apply and highly stable with time.

### BACKGROUND OF THE INVENTION

The present inventors made an invention (as disclosed in WO 01/15658) relating to a cosmetic comprising methyltrimethicone, hereinafter referred to as M3T. The invention was intended to solve problems of cyclic silicones widely used for cosmetics such as octamethylcyclotetrasiloxane. The problems were those occurring in cosmetic production processes and a sensory problem, i.e., dry feel. The problems including the dry feel problem were indeed significantly resolved, but the cosmetic still needs improvements in some properties such as a comfortable feel to apply to the skin. Moreover, there still was room for improvement in an emulsion or solid type cosmetic comprising M3T in sensory feel and stability. Therefore, an object of the present invention is to solve these problems and to provide a further improved cosmetic.

EP 0 915 123 A1 discloses cosmetic preparations comprising volaltile methyl siloxanes such as tetraquistrimethylsiloxysilane and/or M3T, further volatile solvents such as octamethylcyclotetrasiloxane and optional other excipients. The compositions are indicated to be able to be used alone or blended with other cosmetic fluids to form a variety of over-the-counter personal care products.

US 2 567 110 A discloses the preparation of organopolysiloxanes by the reaction of alkali metal salts of an organo silanol with a halosilane. The preparation of tetraquistrimethylsiloxysilane and M3T is described in Examples 20 and 21, respectively of the aforementioned U.S. application.

### DISCLOSURE OF THE INVENTION

The present invention is a process for making a cosmetic comprising tetraquistrimethylsiloxysilane represented by the formula (1), which process comprises subjecting tetraalkoxysilane represented by the following formula (A) and hexamethyldisiloxane represented by the formula (B),

Si(OR)₄ (A)

wherein R is a monovalent hydrocarbon group having 1 to 10 carbon atoms,

(CH₃)₃SiOSi(CH₃)₃ (B)

to a reaction according to a method comprising the steps of
(1) mixing 2 to 10 moles of hexamethyldisiloxane and 0.01 to 0.5 moles of an acid catalyst and adjusting the temperature to a temperature ranging from 0°C to below 30 °C,
(2) adding 1 mole of tetraalkoxysilane to the mixture obtained in the step (1),
(3) adding 2.5 to 10.0 moles of water to the mixture obtained in the step (2) and subjecting to a reaction for 30 minutes to 5 hours while keeping the temperature of from 0 °C to below 30°C, and
(4) subjecting to a reaction for 30 minutes to 5 hours at a temperature of from 30 °C to 100 °C.

[(CH₃)₃SiO]₄Si (1).

A cosmetic comprising the aforesaid tetraquistrimethylsiloxysilane, hereinafter referred to as M4Q, is described in Japanese Patent Application Laid-Open No.10-176059, Japanese Patent Application Laid-Open No.2000-281796, and WO 00/64401.

However, these documents mention M4Q as an equivalent of the aforesaid cyclic silicones, as a mere example of the silicones having a low boiling point, or as one of the MQ type resins. These documents neither describe nor suggest making use of advantages of M4Q over M3T or the cyclic silicones to use M4Q for cosmetics. Further, these documents do not mention anything about irritant impurities possibly contained in industrially manufactured M4Q.

Preferably, the aforesaid cosmetic further comprises methyltrimethicone represented by the formula (2) and/or a solvent volatile at 25°C and 1 atm.

[(CH₃)₃SiO]₃SiCH₃ (2)

Preferably, the volatile solvent is at least one selected from the group consisting of linear dimethylpolysiloxane having 4 or 5 silicon atoms, cyclic dimethylpolysiloxane having 4 to 6 silicon atoms, monohydric alcohols having 1 to 3 carbon atoms, and hydrocarbons having 10 to 16 carbon atoms.

Preferred embodiments of the present cosmetic are in the form of a W/O type emulsion cosmetic, a cleansing agent, a skin cleaner, a cosmetic comprising a pigment, an oil-based cosmetic, and a solid makeup cosmetic. Preferably, these further comprise a UV-ray protective component.

The cosmetic obtained by the aforesaid method has a content of 1,1,1,5,5,5-hexamethyl-3-trimethylsiloxy-3-hidroxy-trisiloxane represented by the following formula (3)

[(CH₃)₃SiO]₃SiOH (3)

at most 1 mass % based on the total mass of the cosmetic.

### PREFERRED EMBODIDMENTS OF THE INVENTION

The present cosmetic is characterized in that it comprises tetraquistrimethylsiloxysilane (M4Q) represented by the following formula (1) and prepared as described hereinabove.

[(CH₃)₃SiO]₄Si (1)

M4Q is chemically inert and very safe. It is comfortable to apply to the skin without giving dry feel or dryness peculiar to cyclic silicones.

M4Q has a boiling point of 222°C at atmospheric pressure. It is less volatile than M3T having a boiling point of 190 °C at atmospheric pressure. M4Q has a viscosity of about 3.1 mm²/s at room temperature. The term "volatile" as used herein means being volatile at 25°C and 1 atm. M4Q can be incorporated in a cosmetic in an amount of from 0.1 to 90 mass%. Preferably, the amount is adjusted according to a combination with other components and the form of a cosmetic. If the amount of M4Q is below the aforesaid lower limit, advantages of M4Q may not be fully utilized. In contrast, a cosmetic containing M4Q in more than the aforesaid upper limit may be too oily.

By using a mixture of M4Q with M3T, a cosmetic can be obtained which has a desired volatility to make the skin feel more comfortable. The mixing ratio of M4Q to M3T can be adjusted according to the form of a cosmetic or the desired volatility but, preferably, ranges from 5:95 to 90:10 (mass %). In the aforesaid range, a desired volatility and oiliness can be attained, and a cosmetic having excellent safety can be obtained. In a cosmetic base, for example, the ratio ranges from 60:40 to 40:60.

The total amount of M4Q and M3T preferably ranges from 0.1 to 90 mass%, more preferably from 0.5 to 60 mass%, based on the total mass of the cosmetic. In the aforesaid range, a user can enjoy suitable volatility controlled with M4Q and M3T.

Besides M3T, various kinds of volatile solvents can be mixed with M4Q to attain desired volatility and to reduce oiliness of M4Q to thereby prepare a cosmetic which gives fresh feel to the skin when applied to the skin. For example, a combination with a lower alcohol such as ethanol and isopropanol is preferred. Particularly, ethanol can make the skin feel fresh by forming an azeotrope with M4Q.

Meanwhile, a combination with at least one selected from the group consisting of isododecane, isodecane, isohexadecane, isoparaffin, volatile linear silicone, and terpene is preferred. Among these, each isododecane, isodecane, and isohexadecane evaporates fast and a combination with M4Q enables one to make a cosmetic which continues evaporating. It should be noted that each isododecane, isodecane, and isohexadecane irritates the skin when they seal the skin and, therefore, it is preferred to devise a formulation to prevent such sealing. The aforesaid volatile solvent is mixed with M4Q, optionally a mixture of M4Q with M3T, in a mass ratio preferably of from 10:90 to 90:10.

By using M4Q in combination with a conventional volatile silicone, an evaporation rate can be controlled or sensory properties can be modified. Examples of the conventional volatile silicone include cyclic dimethylpolysiloxane having 4 to 6 silicon atoms in an amount not to give dry feel, and volatile linear dimethylpolysiloxane having 4 or 5 silicon atoms. Examples of the cyclic dimethylpolysiloxane are octamethylcyclotetrasiloxane, decamethylcyclopentasiloxane, and dodecamethylcyclohexasiloxane. These may be used in an amount as defined above for the volatile solvent.

The aforesaid M4Q, when used together with an emulsifier, gives an emulsion type cosmetic which is highly stable with time. The emulsion type cosmetics include an O/W type and a W/O type cosmetic. A suitable type can be selected according to the intended use of a cosmetic and the content of oil agents.

Examples of the emulsifier include nonionic silicone surfactants branched-polyglyceryl-modified silicone, branched-polyether-modified silicone, alkyl/polyether-comodified silicone, polyether-modified silicone, crosslinked-polyglyceryl-modified silicone, saccharide-modified silicone, glyceryl-modified silicone, and a mixture thereof, among which branched-polyglyceryl-modified silicone, branched-polyether-modified silicone, alkyl/polyether-comodified silicone, polyether-modified silicone, crosslinked-polyglyceryl-modified silicone, and a mixture thereof are preferred.

These emulsifiers have good compatibility with a volatile silicone used in the present invention and are useful to form a stable emulsion. Among the emulsifiers, the one having a HLB of 6 or smaller is particularly preferred to prepare a W/O type emulsion cosmetic. A combination with another emulsifier may also be used as far as a HLB as a whole is 6 or smaller. The emulsifier may be used preferably in an amount of from 0.05 to 20 mass%, more preferably from 0.1 to 10 mass % based on a total mass of the cosmetic. Details of these compounds are described in publications, for example, in Japanese Patent Application Laid-Open No.2002-179548, Japanese Patent Application Laid-Open No.2002-179797, Japanese Patent Application Laid-Open No.2002-179798, Japanese Patent Application Laid-Open No.2002-154917, Japanese Patent Application Laid-Open No.7-252122, Japanese Patent Application Laid-Open No.9-175928, Japanese Patent Publication 44-4679, Japanese Patent Application Laid-Open No.57-14797, Japanese Patent Application Laid-Open No.55-18424, Japanese Patent Application Laid-Open No.10-182354, Japanese Patent Application Laid-Open No.2001-2555, Japanese Patent Application Laid-Open No.2000-63233, Japanese Patent Application Laid-Open No.6-145023, Japanese Patent Application Laid-Open No.7-187945, Japanese Patent Application Laid-Open No.5-112424, Japanese Patent Application Laid-Open No.9-71504.

Another advantage of M4Q is that it is a good solvent. For example, various kinds of silicone materials widely used for cosmetics are dissolved in M4Q at a high concentration. Examples of the silicone materials preferably used together with M4Q include silicone resins, silicone-modified organic thickeners, fluorine-modified silicone resins, polyamide-modified silicone, acrylated silicone, alkyl/acrylic-comodified silicone, silicone-modified pullulan, urethane-modified silicone, and alkyl-modified silicone.

Conventionally, these silicone materials were used by dissolving in a dimethylpolysiloxane having a low viscosity or a cyclic silicone. They can dissolve in M4Q at a higher concentration which allows one to reduce the amount of carry over components and accordingly an increased freedom of formulation. These resin components are used preferably in an amount of from 0.3 to 20 mass % based on a total mass of a cosmetic. In the range, a cosmetic having a good durability can be prepared by making use of qualities of the silicone resin.

These materials are silicone compounds prepared by modifying a silicone chain or silicone resin with various kinds of modifying groups. Details of these are described in publications, for example, Japanese Patent Application Laid-Open No.61-161211, Japanese Patent Application Laid-Open No.4-312511, Japanese Patent Application Laid-Open No.2-42008, Japanese Patent Application Laid-Open No.9-25218, Japanese Patent Application Laid-Open No.2003-55155, Japanese Patent Application Laid-Open No.59-20360, Japanese Patent Application Laid-Open No.9-110633, Japanese Patent Application Laid-Open No.6-9332, Japanese Patent Application Laid-Open No.10-29921, French Patent 2708199, Japanese Patent Application Laid-Open No.3-264510, Japanese Patent Application Laid-Open No.4-66513, Japanese Patent Application Laid-Open No.5-262987, and Japanese Patent Application Laid-Open No.5-194147.

The aforesaid excellent property of M4Q as a solvent is suitable for dissolving greasy dirt. That is, M4Q is suitable for a cleansing agent to remove an oil-based cosmetic and a cleaner to remove sebaceous dirt. In a preferred embodiment of the invention, the cleansing agent comprises M4Q and a nonionic surfactant. M4Q is incorporated preferably in an amount of from 1 to 50 mass % based on the total mass of the cleansing agent. In this range, effective and efficient cleansing performance can be attained.

As the nonionic surfactant, any nonionic surfactant generally used for a cosmetic may be used. Particularly, a silicone surfactant is preferably used because of its good compatibility with M4Q. Examples of the silicone surfactant include those having at least one kind of a modifying group selected from the group consisting of polyoxyalkylene groups, partially substituted or unsubstituted alkyl groups having 2 to 30 carbon atoms, alcoholic hydroxyl groups, phenyl groups, glyceryl groups, polyglyceryl groups, saccharide residues, oxazoline groups, and perfluoropolyether, bonded to a dimethylpolysiloxane chain, and also having a modifying pendant or terminal group or block unit containing a hydrophilic group, such as polyoxyalkylene groups, glyceryl groups, saccharide residues, perfluoropolyether groups, and alcoholic hydroxyl groups, as an essential constituent. For example, the following compounds may be used: polyether-modified organopolysiloxanes (also called polyoxyalkylene-modified silicones, polyether-modified silicones, or polyether-modified siloxanes), alkyl/polyoxyalkylene-comodified silicones (also called polyetheralkyl-comodified siloxanes), fluorinated dimethiconols, perfluoroalkyl/polyoxyalkylene-comodified silicones, perfluoroalkoxy/polyoxyalkylene-comodified silicones, glyceryl-modified silicones, polyglyceryl-modified silicones, perfluoroalkyl/polyglyceryl-comodified silicones, and glycosyl-modified silicones. Examples of such polyether-modified organopolysiloxanes include KF351, KF6011, KF6012, KF6015, KF6017, KF6026, and FPD6131, produced by Shin-Etsu Chemical Co., Ltd.

Nonionic surfactants such as sorbitan fatty acid esters, glycerin fatty acid esters, polyglycerin fatty acid esters, propylene glycol fatty acid esters, polyethylene glycol fatty acid esters, sucrose fatty acid esters, polyoxyethylene alkylethers, polyoxypropylene alkylethers, polyoxyethylene alkylphenylether, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanolether, polyoxyethylene phytosterolether, polyoxyethylene cholestanolether, polyoxyethylene cholesterylether, alkanolamide, sugar ethers, and sugar amides are also be preferred. The nonionic surfactant is incorporated preferably in an amount of from 0.1 to 25 mass %, particularly from 0.5 to 10 mass % based on the total mass of the cleansing agent.

To prepare an emulsion type cleansing agent by mixing water, the emulsion is preferably of O/W type. For the cleansing agent, nonionic surfactant having a HLB of 10 or larger is particularly preferred because an effective cleansing performance is attained.

The cleansing agent may not contain water. An oil type cleansing agent without water can be made to be transparent by selecting a refractive index of the oil agent.

The cleansing agent may be in the form of liquid, spray, sheet, mist, or mousse, used for face, the skin surrounding the eyes, hair or body.

The cleaner which can be prepared by the process of the present invention will be explained. The cleaner is to remove makeup and dirt on the skin or hair at a site without facilities for wet cleaning (Japanese Patent Application Laid-Open No.5-201851). Conventionally, cyclic silicones were used for this purpose. However, as described above, the cyclic silicones causes dry feel and do not remove sebum very well. In contrast, M4Q does not cause dry feel and removes sebum well. It gives a cleaner with good usability without irritating or damaging the skin.

In a preferred embodiment of the invention, the present skin cleaner comprises M4Q and methyl phenyl polysiloxane. As the methyl phenyl polysiloxane, any methyl phenyl polysiloxane generally used for cosmetics can be used, but those which are liquid at room temperature and atmospheric pressure are preferred. The methyl phenyl polysiloxane is used preferably in an amount of from 0.5 to 25 mass% based on the total mass of the cleaner. In this range, improved sensory properties can be attained with only a little oiliness remaining after use of the cleaner.

Preferably, the cleaner further comprises a solvent volatile at 25°C and 1 atm with a boiling point of 225°C or lower at 1 atm. Examples of the volatile solvent include aforesaid volatile silicones such as methyl trimethicone, cyclic silicones, linear silicone having a low molecular weight; lower alcohols such as ethyl alcohol, isopropyl alcohol, and propyl alcohol; and light liquid paraffin such as isododecane, among which methyl trimethicone and ethyl alcohol are preferred because they are very safe and have a good miscibility with sebum substance. Water can be incorporated. However, to prevent sebum or dirt from redepositing on the skin, the amount of water is preferably at most 10 mass % based on a total mass of the cosmetic, more preferably 0. The cleaner containing 5 to 70 mass % of M4Q, 0.5 to 25 mass% of methylphenylpolysiloxane and 25 to 94.5 mass% of a volatile solvent can attain high cleaning performance.

The cleaner may be used as a cleansing agent or a dry shampoo in the form preferably of liquid, spray or sheet.

M4Q disperses pigments very well and is advantageously used as a dispersion medium in a cosmetic comprising a pigment. Conventionally, ester oils or cyclic silicones were used as dispersion medium in a method, so-called color base method, where a paste comprising pigment is prepared for each color and a color of a product is made by blending two or more of the paste. An advantage of this method is that it is easy to match color. However, the ester oils are degraded by mechanical energy applied in a dispersion process to cause odor or discoloration. The cyclic silicones are resistant to the mechanical force but not such a good medium to disperse pigments uniformly with ease. In contrast, M4Q is found to be resistant to mechanical pulverization process and disperses coloring pigments very well and, therefore, it is suitable as a dispersion medium. The present inventors have dispersed various coloring pigments in M4Q and incorporated them in cosmetics. The cosmetics obtained were colorful with a high chromaticness.

As the coloring pigment, any pigments commonly used in cosmetics may be used, regardless of the shape (spherical, rod-like, acicular, tubular, irregular, scaly or spindle forms), particle size (size of fume, fine particles or pigment grade), and particle structure (porous and non-porous).

Examples of coloring pigments include inorganic red pigments such as iron oxide, iron hydroxide, and iron titanate, inorganic brown pigments such as γ-iron oxide; inorganic yellow pigments such as iron oxide yellow and loess; inorganic black pigments such as iron oxide black, (Ti₂O₃)_{z}, and carbon black, inorganic violet pigments such as manganese violet and cobalt violet, inorganic green pigments such as chromium hydroxide, chromium oxide, cobalt oxide, and cobalt titanate, inorganic blue pigments such as Prussian blue and ultramarine blue, lakes of tar pigments, lakes of natural dyes, and synthetic resin powder complexes thereof.

Examples of tar pigments include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207; and natural pigments such as carminic acid, laccaic acid, carthamin, brazilin, and crocin. Among these, tar pigments, lakes thereof and natural pigments are preferred.

Those coloring pigments are preferably surface-treated in a conventional method, e.g., treatment with a fluorine compound, preferably perfluoroalkylphosphate, perfluoroalkylsilane, perfluoropolyether, fluorosilicone, and fluorinated silicone resin, treatment with silicone, e.g., methylhydrogenpolysiloxane, dimethylpolysiloxane, or vapor phase treatment with tetramethyltetrahydrogen cyclotetrasiloxane, pendant treatments, i.e., addition of an alkyl chain after vapor phase treatment with silicone, treatment with a silane coupling agent, treatment with a titanium coupling agent, treatment with silane, preferably alkylsilane or alkylsilazane, treatment with an oil agent, treatment with N-acylated lysine, treatment with polyacrylic acid, treatment with a metal soap, preferably stearic acid or myristic acid salts, treatment with an acrylic resin, treatment with metal oxide, treatment with gelatin, and treatment with deoxyribonucleic acid. It is more desirable to apply a combination of plural treatments selected from the aforementioned treatments.

Surface treatments particularly suited for dispersion in M4Q include treatment with Nε-lauroyl-L-lysine, treatment with alkoxysilane and pendant treatment, among which treatment with Nε-lauroyl-L-lysine and treatment with alkoxysilane are preferred because of their excellent productivity and cost performance.

In a dispersion liquid, M4Q is contained preferably in an amount of from 20 to 90 mass % based on the total mass of the dispersion liquid. To improve dispersion of the coloring pigment, a surfactant, particularly, a silicone surfactant having a structure similar to M4Q is preferably used.

Examples of the silicone surfactant include silicones having at least one kind of a modifying group selected from the group consisting of polyoxyalkylene groups, partially substituted or unsubstituted alkyl groups having 2 to 30 carbon atoms, alcoholic hydroxyl groups, phenyl groups, glyceryl groups, saccharide residues, oxazoline groups, and perfluoropolyether, bonded to a dimethylsiloxane chain, and also having a modifying pendant or terminal group or block unit containing a hydrophilic group, such as polyoxyalkylene groups, glyceryl groups, saccharide residues, perfluoropolyether groups, and alcoholic hydroxyl groups, as an essential constituent. For example, the following compounds may be used: polyether-modified organopolysiloxanes (also called polyoxyalkylene-modified silicones, polyether-modified silicones, or polyether-modified siloxanes), alkyl/polyoxyalkylene-comodified silicones (also called polyetheralkyl-comodified siloxanes), fluorinated dimethiconols, perfluoroalkyl/polyoxyalkylene-comodified silicones, perfluoroalkoxy/polyoxyalkylene-comodified silicones, glyceryl-modified silicones, perfluoroalkyl/polyglyceryl-comodified silicones, and glycosyl-modified silicones. Among these silicone surfactants, ones having an HLB ranging from 1 to 12 are desirable due to their excellent compatibility with M3T, and those having an HLB ranging from 1 to 9 are most desirable. Examples of such polyether-modified organopolysiloxanes include KF6012, KF6015, KF6017, KF6026, and FPD6131, produced by Shin-Etsu Chemical Co., Ltd.

Another dispersant, preferably a nonionic surfactant, is used. Examples of the nonionic surfactant include sorbitan fatty acid esters, glycerin fatty acid esters, polyglycerin fatty acid esters, propylene glycol fatty acid esters, polyethylene glycol fatty acid esters, sucrose fatty acid esters, polyoxyethylene alkylethers, polyoxypropylene alkylethers, polyoxyethylene alkylphenylether, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanolether, polyoxyethylene phytosterolether, polyoxyethylene cholestanolether, polyoxyethylene cholesterylether, alkanolamide, sugar ethers, or sugar amides. The surfactant is contained in a dispersion liquid preferably in an amount of from 0.1 to 20 mass %, particularly 0.5 to 15 mass% based on the total mass of the dispersion liquid.

The dispersion liquid is prepared by dispersing the aforesaid components in a mill. It is preferred to prepare a dispersion liquid for each color and blend a plurality of the dispersion liquids into a desired color. Dispersion may be carried out with a wet-medium mill such as a beads mill or Micros, ex Nara Machinery Co., Ltd., a roll mill, a jet mill, a ultrasonic grinder mill, or a high-pressure jet- mill such as Ultimaizer System, ex Sugino Machine Ltd., a disper mill, a high speed homogenizer, or a homogenizer. Among these, the wet-medium mill attains a high degree of dispersion to form a stable dispersion liquid with an excellent productivity. The wet-medium type mill may be of a vertical or horizontal structure and may be a continuous or a batch type. Using the mill, M4Q and a pigment, optionally a dispersant and an oil agent, are mixed and milled. Conditions for milling depend on a capacity of an individual mill. In order to ensure lot-to-lot reproducibility of color, it is useful to make a graph showing milling time or the number of milling versus average particle size or color and to select conditions where the graph indicates a steady state.

Examples of cosmetics for which M4Q is a suitable dispersion medium of pigment include makeup products, particularly lipstick, eyeliner, mascara, eyebrow, and eye shadow, nail color, foundation, concealer, cheek and hair dye.

An oil-based cosmetic can be obtained by mixing an oil agent which is solid or pasty at 25 °C in addition to the aforesaid various pigments. It is known that volatile silicones are incorporated in an oil-based cosmetic such as lipstick in order to prevent color transfer or to improve durability (Japanese Patent Application Laid-Open No. 8-92036, Japanese Patent Application Laid-Open No. 3-77162, Japanese Patent Application Laid-Open No. 10-29915). However, as described above, the volatile silicones, particularly cyclic silicones, give a user dry feel on or after application to the skin. In contrast, M4Q does not give dry feel. In addition, M4Q has lower volatility and better dispersion property than the aforesaid cyclic silicones, so that it gives oil-based cosmetics showing excellent gloss and colorfulness.

In a preferred embodiment of the invention, the oil-based cosmetic comprises M4Q, a pigment, and polybutene. M4Q is contained preferably in an amount of from 0.1 to 60 mass %, more preferably from 1 to 50 mass % based on the total mass of the oil-based cosmetic. In this range, improved stability, color-transfer prevention, and improved colorfulness of the cosmetic can be attained.

The pigment is contained preferably in an amount of from 0.1 to 50 mass%, more preferably from 0.5 to 35 mass % based on a total mass of the cosmetic. In this range, stability of the cosmetic and desirable feel to the touch of the cosmetic can be attained.

Polybutene obtained by copolymerizing isobutene with n-butene is preferred and one in the form of liquid or paste at room temperature is more preferred. Such a polybutene have an average molecular weight preferably ranging from 500 to 2700, more preferably from 800 to 1200. Polybutene with a molecular weight below 500 may cause a cosmetic to be unstable at a high temperature. Polybutene with a molecular weight above 2700 may be tacky to spoil sensory property of a cosmetic. The polybutene used in the present invention may be hydrogenated to improve stability or purified. The polybutene is contained preferably in an amount of from 2 to 40 mass % based on a total mass of the oil-based cosmetic. If it is contained below 2 mass %, sustained-release of M4Q may not be attained. If contained above 40 mass %, a cosmetic may be tacky to have worse feel to the touch.

The oil-based cosmetic can be prepared by any known method. It may be prepared, for example, with a dispersing apparatus such as a roller mill or a beads mill. Alternatively, a color-base may be prepared followed by mixing a plurality of the color-base to prepare the oil-based cosmetic. Because M4Q is volatile, a sealable container should be used.

The oil-based cosmetic is suitably used as a lipstick, foundation, eye shadow, eyeliner, mascara, cheek, concealer, sunscreen, and makeup base.

By mixing water in addition to the aforesaid pigment and oil agent with the aid of a surfactant, a solid makeup cosmetic can be prepared. In the present invention, a preferred embodiment of the makeup cosmetic is shown below, wherein each amount represented in mass% is based on the total mass of the cosmetic.
(A) 10 to 30 mass % of M4Q,
(B) 5 to 10 mass % of at least one polyol compound selected from the group consisting of polyhydric alcohols and saccharides,
(C) 1.5 to 3.5 mass % of a surfactant selected from the group consisting of sorbitan fatty acid esters, polyglycerin fatty acid esters, sucrose fatty acid esters, and polysaccharide alkyl ethers,
(D) 3 to 7 mass % of an oil agent which is solid or pasty at 25 °C,
(E) 30 to 45 mass % of a pigment, and
(F) 5 to 25 mass % of purified water.

Examples of polyol compounds (B) include glycols and polysaccharides, for example, ethylene glycol, propylene glycol, butylene glycol, diethylene glycol, triethylene, di propylene glycol, glycerol, diglycerol, sorbitol, maltitol, trehalose, raffinose, xylitol, mannitol, hyaluronic acid or a salt thereof, trehalose derivatives, raffinose derivatives, polyethylene glycol, and polyglycerol.

Examples of the trehalose derivatives and raffinose derivatives include alkyl ether derivatives and alkenyl ether derivatives of theses saccharide, among which alkyl ether derivatives having 1 to 24 carbon atoms and alkenyl ether derivatives having 3 to 24 carbon atoms are preferred. Examples of polysaccharides include carrageenan, agar-agar, guar gum, locust bean gum, gum Arabic, tragacanth gum, pectin, xanthan gum, dextran and cellulose. The polyol is contained in an amount of from 5 to 10 mass % based on the total mass of the solid makeup cosmetic. If the polyol is contained in less than the aforesaid lower limit, the skin may be strained after a cosmetic is applied. A cosmetic containing the polyol in more than the aforesaid upper limit may be too tacky.

In the present solid cosmetic, the aforesaid various kinds of surfactants (C) can be used. Among those, at least one selected from the group consisting of sorbitan isostearate and sorbitan monooleate is preferably used. The surfactant is contained in an amount of from 1.5 to 3.5 mass % based on the total mass of the solid makeup cosmetic. If the content is below the aforesaid lower limit, water resistance and sebum resistance may be lower. A cosmetic containing the polyol in more than the aforesaid upper limit may be tacky.

Examples of the solid or pasty oil agent (D) include resins and thickeners that are dissolved in an oil agent to be in the form of paste at room temperature as well as oil agents and resins commonly used for cosmetics.

Typical examples include jojoba wax, carnauba wax, candelilla wax, rice bran oil, shellac, lanoline, beeswax, bleached beeswax, ozokerite, ceresin, paraffin, microcrystalline wax, vaseline, lauric acid, myristic acid, palmitic acid, stearic acid, behenic acid, 12-hydroxystearic acid, undecylenic acid, lauryl alcohol, myristyl alcohol, cetyl alcohol, stearyl alcohol, oleyl alcohol, behenyl alcohol, batyl alcohol, chimyl alcohol, oleyl glyceryl ether, isostearyl glyceryl alcohol, ethyl stearate, butyl stearate, myristyl myristate, myristyl stearate, cetyl ricinolate, glyceryl triundecylenate, pentaerythritol tetramyristate, cetyl lactate, octyl hydroxystearate, cholesteryl stearate, cholesteryl isostearate, cholesteryl hydroxystearate, chlesteryl oleate, dihydroxychelsteryl oleate, phytoseryl isostearate, polyethylene glycol, polyglycerol, palm oil, coconut oil, beef tallow, hydrogenated oil, horse fat, shear butter, egg yolk oil; and resins such as polyethylene, polypropylene, polytetrafluoroethylene, acrlic silicone, acrylic polymer or a salt thereof, acrylic acid/methacrylic acid copolyemr or a salt thereof, and polyvinylpyrrolidone.

The solid or pasty oil agent is contained in an amount of from 3 to 7 mass% based on the total mass of the cosmetic. If the content is below the aforesaid lower limit, water resistance and sebum resistance may not be attained. If the content exceeds the aforesaid upper limit, a cosmetic may be difficult to remove and may have worse feel to the touch.

The pigment (E) is contained in an amount of from 30 to 45 mass % based on the total mass of the solid makeup cosmetic. If it is contained below the aforesaid lower limit, stability of a cosmetic may not be satisfactory. Accordingly, it is preferred to make the content higher than the aforesaid lower limit by a combined use with an oily gelling agent. If contained in more than the aforesaid upper limit, a cosmetic may not have a good feel to the touch and may be difficult to remove.

The pigment may either hydrophilic or hydrophobic as far as it is commonly used for cosmetics but, preferably, hydrophobic ones are used. Examples of the commonly used pigments include inorganic powder, organic powder, surface active, metal salt powder, colored pigments, pearl pigments, metallic powder pigments, and natural colors.

In addition to the aforesaid coloring pigments, tar pigments, and natural dyes, examples of the pigments include inorganic powder such as such as titanium oxide, zirconium oxide, pigment grade zinc oxide, cerium oxide, magnesium oxide, barium sulfate, calcium sulfate, magnesium sulfate, calcium carbonate, magnesium carbonate, talc, mica, kaolin, cericite, muscovite, synthetic mica, phlogopite, lepidolite, biotite, lithia mica, silicic acid, silicic anhydride, aluminum silicate, magnesium silicate, aluminum magnesium silicate, calcium silicate, barium silicate, strontium silicate, metal salts of tungstenic acid, hydroxyapatite, vermiculite, higilite, bentonite, montmorillonite, hectolitre, zeolite, ceramics powder, dibasic calcium phosphate, alumina, aluminum hydroxide, boron nitride, and silica, titanium oxide fine power, low-dimensional titanium oxide fine powder, zinc oxide fine powder, cerium oxide fine powder, and cerium oxide fine powder with repressed activity;organic powder such as organic powder such as polyamide powder, polyester powder, polyethylene powder, polypropylene powder, polystyrene powder, polyurethane powder, benzoguanamine powder, polymethylbenzoguanamine powder, polytetrafluoroethylene powder, polymethylmethacrylate powder, cellulose powder, silk powder, nylon powder such as Nylon 12 and Nylon 6, silicone powder, silicone gum powder, silicone elastomer spherical powder, polymethylsilsesquioxane spherical powder, polyalkylsilsesquioxane powder, styrene/acrylic acid copolymer, divinylbenzene/styrene copolymer, vinyl resin, urea resin, phenol resin, fluororesin, silicone resin, acrylic resin, melamine resin, epoxy resin, polycarbonate resin, microcrystalline fiber powder, starch powder, and lauroyl lysine; surface active metal salt powders (metal soaps) such as zinc stearate, aluminum stearate, calcium stearate, magnesium stearate, zinc myristate, magnesium myristate, zinc cetyl phosphate, calcium cetyl phosphate, and zinc/sodium cetyl phosphate; and pearl pigments such as titanium oxide-coated mica, titanium oxide-coated mica, bismuth oxychloride, titanium oxide-coated bismuth oxychloride, titanium oxide- coated talc, fish scales, and titanium oxide-coated colored mica. These pigements may be surface treated as mentioned above.

Purified water (F) is contained in an amount of from 5 to 25 mass % based on the total mass of solid makeup cosmetic. If water is contained in less than the aforesaid lower limit, a cosmetic may lack fresh feel to the touch. A cosmetic containing purified water in more than the aforesaid upper limit may not be stable. As the purified water, ultra pure water, mineral water, and hot spring water can be used.

The solid makeup cosmetic obtained appears solid and flows when scrubbed. It is served for use packed in a sealable container. The present solid makeup cosmetic can be used for any application and suitably used as a makeup product such as foundation, concealer, eye shadow or lipstick. As skincare products and UV-ray protective products, it is suitable as a sunscreen agent for everyday use.

As described above, M4Q is suitable to prepare various kinds of cosmetics with excellent qualities. However, it was found that a by-product of the M4Q production process, 1,1,1,5,5,5-hexamethyl-3-trimethylsiloxy-3-hydroxytrisiloxane, hereinafter referred to as M3Q(OH), causes a big sensory problem. That is, 1,1,1,5,5,5-hexamethyl-3-trimethylsiloxy-3-hydroxytrisiloxane gives the skin slippery feel when it is present in liquid state on the skin, which is not so desirable in a cosmetic; About 5 after the application, it makes the skin feel strained; In about 10 minutes, it completely evaporates to restore the skin to its original state. Thus, the skin undergoes a very big sensory change.

As a result of extensive studies, it has been found that a preferred concentration of 1,1,1,5,5,5-hexamethyl-3-trimethylsiloxy-3-hydroxy-trisiloxane is at most 1.0 mass %, more preferably at most 0.5 mass %, most preferably at most 0.35 mass % based on the total mass of a cosmetic.

Further, to make the concentration of 1,1,1,5,5,5-hexamethyl-3-trimethylsiloxy-3-hydroxy trisiloxane in the aforesaid range, a preferred concentration of the same in M4Q is at most about 3.0 mass %, more preferably at most about 1.0 mass %, most preferably at most about 0.5 mass %.

However, 1,1,1,5,5,5-hexamethyl-3-trimethylsiloxy-3-hydroxytrisiloxane has a boiling point close to that of M4Q, the concentration thereof in M4Q exceeds 3 mass % depending on the production process of M4Q, so that much time, energy and cost is required to remove it by distillation. Therefore, studies were made in pursuit of a method of preparing M4A wherein the residual amount of 1,1,1,5,5,5-hexamethyl-3-trimethylsiloxy-3-hydroxytrisiloxane is minimal.

As a result, M4Q made by the following method contains such a small amount of 1,1,1,5,5,5-hexamethyl-3-trimethylsiloxy-3-hydroxytrisiloxane as the aforesaid 3 mass % without purification process, for example, distillation, which M4Q is suitable for cosmetics.

A method of preparing M4Q by subjecting the tetraalkoxysilane represented by the formula (A) and hexamethyldisiloxane represented by the formula (B) to a reaction according to a method comprising the steps of
(1) mixing 2 to 10 moles of hexamethyldisiloxane and 0.01 to 0.5 mole of a catalyst, per mole of tetraalkoxysilane which is to be added in step (2), and adjusting the temperature of the resulting mixture to a temperature of from 0 °C to below 30 °C,
(2) adding 1 mole of tetraalkoxysilane to the mixture obtained in the step (1),
(3) adding 2.5 to 10.0 moles of water to the mixture obtained in the step (2) and subjecting to a reaction for 0.5 to 5 hours while keeping the temperature of from 0 °C to below 30 °C, and
(4) subjecting to a reaction for 0.5 to 5 hours at a temperature of from 30 °C to 100 °C.

The purity of M4Q can be determined by, for example, a peak area ratio in gas chromatogram, details of which will be described in Examples herein below.

In the formula (A), R is a monovalent hydrocarbon group having 1 to 10 carbon atoms, for example, ethyl, n-propyl, isopropyl, n-butyl, isobutyl, sec-butyl, tert-butyl, n-hexyl, octyl, 2-ethylhexyl, nonyl, and decyl groups. R may be the same with or different from each other. Preferably, R is an ethyl group or an isopropyl group.

The tetraalkoxysilane may be used which is prepared by a well-known method and then purified. Alternatively, a reaction mixture obtained by subjecting tetrachlorosilane and an alcohol to an elimination reaction of hydrochloric acid can be used without purification.

The unpurified tetraalkoxysilane obtained by subjecting tetrachlorosilane and an alcohol to an elimination reaction of hydrochloric acid is generally highly pure, containing alcohol and a small amount of disiloxane as impurities. In the present invention, tetraalkoxysilane with 50 % or higher purity can be used with no problem but, preferably, the purity is 80 % or higher, more preferably 90 % or higher.

In the above method, hexamethyldisiloxane is used in an amount of from 2 to 10 moles, more preferably from 2.5 to 5.0 moles, per mole of tetraalkoxysilane. If the amount of hexamethyldisiloxane is below 2.0 moles, the reaction yield may smaller due to increased residual alkoxy intermediates, such as
1,1,1,5,5,5-hexamethyl-3-trimethylsiloxy-3-methoxytrisilo xane, ({(CH₃)₃SiO}₃SiOCH₃, herein after referred to as M3Q(OMe))and
1,1,1,5,5,5-hexamethyl-3-trimethylsiloxy-3-ethoxytrisiloxane, ({(CH₃)₃SiO}₃SiOCH₂CH₃, herein after referred to as M3Q(OEt)). More than 10.0 moles of hexamethyldisiloxane do not increase the yield but decrease the pot yield, i.e., the ratio of the amount of product to the total amount of raw materials.

In the aforesaid step (1), it is preferred to add 0.5 to 10.0 moles per mole of tetraalkoxysilane of a lower monohydric alcohol having 1 to 6 carbon atoms. Examples of the lower monohydric alcohol having 1 to 6 carbon atoms include methanol, ethanol, n-propanol, isopropanol, n-butanol, isobutanol, pentanol, and hexanol, among which methanol, ethanol, and isopropanol are preferred.

The amount of the lower monohydric alcohol ranges preferably from 0.5 to 10 moles, particularly from 1 to 5 moles per mole of tetraalkoxysilane. If the amount of the lower monohydric alcohol is below 0.5 mole, water, which is essential to hydrolysis reaction, may not be sufficiently dispersed to increase residual reaction intermediates or products having a high boiling point, resulting in a decreased reaction yield. If the amount exceeds 10 moles, the pot yield may be decreased.

When tetramethoxysilane, Si(OCH₃)₄, is to be used, it is preferred to use, for one mole of tetramethoxysilane which is to be added in the step (2), 2.0 to 10.0 moles of hexamethyldisiloxane, 1. 0 to 5.0 moles of the lower monohydric alcohol selected from methanol, ethanol and isopropanol, 0.01 to 0.5 moles of acid catalyst, and 2.5 to 10.0 moles of water, and to perform the water addition step (3) at a temperature of from 0°C to below 30°C for 30 minutes to 5 hours; and then to carry out a hydrolysis and condensation reaction at a temperature of from 30°C to 100°C for 30 minutes to 5 hours.

When the tetraalkoxysilane with R being other than a methyl group, particularly tetraethoxysilane or tetrapropoxysilane, is used, ethanol or propanol is formed as the hydrolysis proceeds. These alcohols improve miscibility of water with alkoxysilane, which allows one to save the use of the lower monohydric alcohol. The synthesis method requiring no lower monohydric alcohol is particularly desired from the viewpoint of pot yield.

Examples of the acid catalyst include sulfuric acid, hydrochloric acid, methane sulfonic acid and trifluoromethane sulfonic acid. Particularly, sulfuric acid and trifluoromethane sulfonic acid are preferred. The catalyst is used in an amount per mole of the tetraalkoxysilane of from 0.01 to 0.5 moles, more preferably from 0.02 to 0.4 moles. If the amount is below 0.01 moles, the reaction time may be longer due to lower reaction rate. If the amount exceeds 0.5 moles, reaction products having high a boiling point increase, resulting in lower reaction yield.

In this reaction, water may be used alone or in a mixture with a lower monohydric alcohol having 1 to 6 carbon atoms. Examples of the lower monohydric alcohol include methanol, ethanol, n-propanol isopropanol n-butanol, and isobutanol. The total amount of the lower monohydric alcohol is preferably in the range as defined above.

In this reaction, water is used in an amount of from 2.5 to 10.0 moles, more preferably from 3.0 to 8.0 moles, per mole of tetraalkoxysilane. If the amount of tetraalkoxysilane is below 2.5 moles, the reaction yield may be smaller due to increased residual alkoxy intermediates such as M3Q(OMe) and M3Q(OEt). If the amount exceeds 10 moles, the reaction yield may not increase but the pot yield decreases.

In this reaction, it is preferred that hexamethyldisiloxane is mixed with the optional lower monohydric alcohol having 1 to 6 carbon atoms first and then the acid catalyst and tetraalkoxysilanes are added. Hexamethyldisiloxane, the alcohol, the acid catalyst and tetraalkoxysilane may be mixed at the same time, but preferably, before adding water, the temperature of the reaction mixture is adjusted to a temperature of from 0°C to below 30°C, more preferably from 0°C to 25°C, particularly 0°C to 15°C. If water is added at a temperature below the aforesaid lower limit, the reaction time may be too long due to a smaller reaction rate. If water is added at a temperature above the aforesaid temperature, the reaction yield of M4Q may be too low.

To increase the yield of M4Q, hexamethyldisiloxane is mixed with the optional lower monohydric alcohol and the temperature of the mixture is adjusted to a temperature ranging from 0°C to below 30°C, preferably from 0°C to 25°C, particularly from 0°C to 15°C. Then, the acid catalyst is added to the mixture in such a manner that the temperature of the mixture does not rise to 30°C or higher, preferably not above 25°C, more preferably not above 15°C and the mixture is stirred at that temperature for 10 minutes to 1 hour. Subsequently, tetraalkoxysilane is added in such a rate that the temperature is kept within the range of from 0°C to below 30°C, preferably from 0°C to 25°C, particularly from 0°C to 15°C. The mixture thus obtained is stirred at that temperature for 10 minutes to 1 hour and then water is added to the mixture.

In this reaction, additional water is added to cause hydrolysis. However, it raises the temperature, so that water is preferably added while cooling the reaction mixture to keep the temperature thereof ranging from 0°C to below 30°C. If the reaction mixture is cooled below 0°C, reaction time may be too long due to slowed reaction. If the temperature of the reaction mixture exceeds 30°C, the reaction yield may be smaller. Preferably, water it added while keeping the temperature of from 0°C to 25°C, preferably from 0°C to 15°C. After the addition of water completes, it is preferred to continue stirring at a temperature of from 0°C to below 30°C for 10 minutes to 5 hours.

After the aforesaid steps, the reaction mixture is heated to a temperature ranging from 30°C to 100°C, preferably from 40°C to 80°C and kept stirred for 30 minutes to 5 hours, preferably from 1 to 3 hours. By doing so, residual alkoxy intermediates such as M3Q(OMe) and M3Q(OEt), and M3Q(OH) decrease, resulting in increased yield of M4Q.

After reaction completes, the reaction mixture is phase separated into an aqueous layer and an organic layer. The aqueous layer is removed and the organic layer is preferably neutralized with an aqueous basic solution such as sodium bicarbonate solution. The organic layer is further washed with water until washing fluid becomes neutral and then subjected to purification by distillation or dehydration with a drying agent such as sodium sulfate anhydride or calcium chloride followed by distillation.

For reference, synthetic methods of prior art are described below. For a synthesis of M4Q, synthetic methods of M3T serve as useful references. The following synthetic methods of M3T are known.
1. Hydrolysis of methyltrichlorosilane and trimethylchlorosilane in the presence of methanol(WO 2001-15658, Japanese Patent Application Laid-Open No. 2002-68930)
2. Reaction of methyltrichlorosilane with hexamethyldisiloxane in the presence of perchloric acid catalyst (Dokl.Akad.Nauk SSSR, 227,607-610(1976)).
3. Reaction of methytriethoxysilane with hexamethyldisiloxane in the presence of an acidic ion exchange resin(J.Organomet.Chem.,340,31-36(1988)).
4. Reaction of methyltrialkoxysilane with hexamethyldisiloxane in the presence of a carboxylic acid and an acid catalyst(Japanese Patent Application Laid-Open No. 11-217389).
5. Reaction of methyltrimethoxysilane with hexamethyldisiloxane in methanol by adding concentrated sulfuric acid and then adding dropwise a mixture of water and methanol (WO2001-15658, Japanese Patent Application Laid-Open No. 2002-68930).

Method 1. has drawbacks that it requires a lot of water and the yield is very low due to low reaction selectivity.

Method 2. is not suitable for industrial production due to difficult handling of perchloric acid catalyst.

Method 3. requires a large excess amount of hexamethyldisiloxane to increase the yield. Another drawback is that a large amount of a reaction intermediate, 1,1,1,3,5,5,5-heptamethyl-3-ethoxytrisiloxane, {(CH3)₃SiO}₂Si(OCH₂CH₃) CH₃, hereinafter referred to as M2T(OEt), remains in the synthesis of methyltrimethicone. To synthesize methyltrimethicone, three trimethylsiloxy groups are to be introduced to a silicon atom, wherein reactivity of the third trimethylsiloxy group is lower than the second one. In the synthesis of tetraquistrimethylsiloxysilane, four trimethylsiloxy groups are to be introduced to a silicon atom, wherein reactivity of the fourth trimethylsiloxy group is considered to be far lower than the third one. It follows that a higher amount of a reaction intermediate, M3Q(OEt), is considered to remain. Therefore, this method is not suitable for preparing highly pure tetraquistrimethylsiloxysilane.

In method 4, a reaction intermediate, M2T(OEt), remains in the synthesis of methyltrimethicone. If tetraquistrimethylsiloxysilane is synthesized in the same manner, a large amount of the intermediate, M3Q(OEt), is considered to remain and therefore this method is not suitable for preparing highly pure tetraquistrimethylsiloxysilane.

In method 5, as in aforesaid method 4, a reaction intermediate, {(CH₃)₃SiO}₂Si(CH₃)OCH₃, referred to as M2T(OEt), remains in the synthesis of methyltrimethicone. If tetraquistrimethylsiloxysilane is synthesized in the same manner, a large amount of the intermediate, M3Q(OEt), is considered to remain and therefore this method is not suitable for preparing highly pure tetraquistrimethylsiloxysilane.

In contrast to the aforesaid conventional methods, in the method of the present invention, a product with an amount of M3Q(OH) of at most about 3 mass % can be obtained even before purification as shown in Tables 1 and 2 in Preparation Examples herein below.

Impurities in M4Q preparations can be analyzed by gas chromatography. An example of the analytical condition is as shown below.
Gas chromatograph: GC14B, ex Shimadzu Corp.
Column packing: SE30, trade name, 3 % of liquid phase
Column: inner diameter of 5 mm, and length of 3 m
Detector : TCD
Oven temperature: kept at 80°C for 1 minute and raised to 300 °C at a ramp rate of 15 °C/min.

In the analysis under the aforesaid conditions, M3Q (OMe) is detected at a retention time of 6.2 min., M3Q (OEt) at 6.3 min., M3Q(OH) at 6.6min., M4Q at 7.5 min., and some high boiling point peaks at 10.5 to 13.0 min.

An example of properties of final product, M4Q, are as shown below.
Appearance : colorless and transparent liquid
Boiling point: 215°C at 1 atm, 89.5 to 90.0°C at 1.3 kPa
Viscosity: 3.1mm²/s at 25°C
Specific gravity: 0.864 at 25°C
Refractive index : 1.387 at 25°C
Freezing point : -70°C or lower

M4Q is suitably incorporated in the aforementioned various cosmetics. In the cosmetic of the present invention, a variety of components that are commonly used in cosmetics can be blended in addition to the aforementioned components, for example, UV-ray protectives, oil agents, antiseptics, perfumes, humectants, salts, solvents, antioxidants, chelating agents, neutralizers, pH regulators, insect repellants, and bioactive components.

In the present invention, inorganic and organic UV-ray protection agents may be used as the UV-ray protective component. Examples of the inorganic ones include metal oxides such as titanium dioxide, titanium monoxide, (Ti₂O₃)_{z}, zinc oxide, cerium oxide, and cerium oxide with suppressed activity; metal hydroxide such as iron hydroxide; metal flakes such as tabular iron oxide and aluminum flakes, and ceramics such as silicon carbide. In particular, it is desirable to use at least one kind selected from fine particle metal oxides or fine particle metal hydroxides with a mean particle size ranging from 5 to 100 nm. They may be surface treated as the aforesaid pigments.

Examples of the organic UV-ray protection agents are as follows: 2-ethylhexyl paramethoxycinnamate (also called octyl paramethoxycinnamate), 2-hydroxy-4-methoxybenzophenone, 2-hydroxy-4-methoxybenzophenone-5-sulfuric acid, 2,2'-dihydroxy-4-methoxybenzophenone, p-methoxyhydrocinnamic acid diethanolamine salt, paraaminobenzoic acid (hereinafter referred to as PABA), ethyldihydroxypropyl PABA, glyceryl PABA, homomenthyl salicylate, methyl-O-aminobenzoate, 2-ethylhexyl-2-cyano-3,3-diphenylacrylate, octyldimethyl PABA, octyl salicylate, 2-phenyl-benzimidazole-5-sulfuric acid, triethanolamine salicylate, 3-(4-methylbenzylidene)camphor, 2,4-dihydroxybenzophenine, 2,2', 4, 4'-tetrahydroxybenzophenone, 2,2'-dihydroxy-4,4-dimethoxybenzophenone, 2-hydroxy-4-N-octoxybenzophenone, 4-isopropyl dibenzoylmethane, 4-tert-butyl-4'-methoxydibenzoylmethane, octyltriazone, 2-ethylhexyl 4-(3,4-dimethoxyphenylmethylene)-2,5-dioxo-1-imidazolidine propionate, polymer derivatives thereof, and silane derivatives thereof.

It is also possible to use an organic UV-ray protection agent encapsulated in polymer powder. The polymer powder may be hollow or not, a mean primary particle size may be in the range of 0.1 to 50µm, and the particle distribution may be broad or sharp. Types of the polymer include acrylic resins, methacrylic resins, styrene resins, polyurethane resins, polyethylenes, polypropylenes, polyethylene terephthalates, silicone resins, nylons, and acrylamide resins. The organic UV-ray protection agent is preferably incorporated in the polymer powder in the range from 0.1 to 30 mass% relative to the powder mass. In particular, it is desirable to use 4-tert-butyl-4'-methoxydibenzoylmethane which is a UVA absorbent.

Among the aforementioned UV-ray protective components, use is preferably made of at least one selected from the group consisting of fine particle titanium dioxide, fine particle zinc oxide, 2-ethylhexyl paramethoxycinnamate, 4-tert-butyl-4'-methoxydibenzoylmethane, and UV absorbents of the benzophenone series, because these are widely used and can be obtained easily and their UV protection effect is high. In particular, it is preferred to use an inorganic one and an organic one in combination. It is also preferred to use a combination of one for UV-A with one for UV-B.

Examples of conventional oil agents include avocado oil, linseed oil, almond oil, Ibota wax, perilla oil, olive oil, cacao butter, kapok wax, kaya oil, carnauba wax, Glycyrrhiza oil, candelilla wax, beef tallow, neat' s-foot oil, beef bone fat, hydrogenated beef tallow, apricot kernel oil, spermaceti wax, hydrogenated oil, wheat germ oil, sesame oil, rice germ oil, rice bran oil, sugar cane wax, sasanqua oil, safflower oil, shear butter, Chinese tung oil, cinnamon oil, jojoba wax, shellac wax, turtle oil, soybean oil, tea seed oil, camellia oil, evening primrose oil, corn oil, lard, rapeseed oil, Japanese tung oil, rice bran oil, germ oil, horse fat, persic oil, palm oil, palm kernel oil, castor oil, hydrogenated castor oil, castor oil fatty acid methylester, sunflower oil, grape oil, bayberry wax, jojoba oil, macadamia nut oil, beeswax, mink oil, cottonseed oil, cotton wax, Japanese wax, Japanese wax kernel oil, montan wax, coconut oil, hydrogenated coconut oil, tri-coconut oil fatty acid glyceride, mutton tallow, peanut oil, lanolin, liquid lanolin, hydrogenated lanolin, lanolin alcohol, hard lanolin, lanolin acetate, isopropyl lanolate, hexyl laurate, POE lanolin alcohol ether, POE lanolin alcohol acetate, polyethylene glycol lanolate, POE hydrogenated lanolin alcohol ether, and egg yolk oil; hydrocarbon oils, e.g., ozokerite, squalane, squalene, ceresin, paraffin, paraffin wax, liquid paraffin, pristane, polyisobutylene, microcrystalline wax, and Vaseline; higher fatty acids, e.g., lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid; higher alcohols, e.g., lauryl alcohol, myristyl alcohol, palmityl alcohol, stearyl alcohol, behenyl alcohol, hexadecyl alcohol, oleyl alcohol, isostearyl alcohol, hexyldodecanol, octyl dodecanol, cetostearyl alcohol, 2-decyltetradecinol, cholesterol, phytosterol, POE cholesterol ether, monostearyl glycerin ether (batyl alcohol), and monooleyl glyceryl ether (cerakyl alcohol); ester oils, e.g., diisobutyl adipate, 2-hexyldecyl adipate, di-2-heptylundecyl adipate, N-alkyl glycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, isononyl isononanate, neopentyl glycol dicaprirate, triethyl citrate, 2-ethylhexyl succinate, amyl acetate, ethyl acetate, butyl acetate, isocetyl stearate, butyl stearate, diisopropyl sebacinate, di-2-ethylhexyl sebacinate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid esters, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate, and diisostearyl malate; and glyceride oils, e.g., acetoglyceryl, glycerol triisooctanoate, glyceryl triisostearate, glyceryl triisopalmitate, glyceryl monostearate, glyceryl di-2-heptylundecanoate, glyceryl trimyristate, and diglyceryl myristyl isostearate.

Silicone oils such as dimethylorganopolysiloxanes, amine-modified organopolysiloxanes, organopolysiloxanes having an alcoholic hydroxyl group, organopolysiloxanes having an alkyl group except methyl or phenyl, and amodimethicone can be preferably used because of their excellent compatibility with M4Q. These silicones may have a silicone chain length ranging from 1 to 30000 as a degree of polymerization.

Fluorine compounds can be incorporated such as fluorine-modified silicones, perfluoropolyethers, fluorinated pitch, perfluorodecaline, fluorocarbons such as perfluorooctane, fluoroalcohols, and perfluoroalkylalkylethers. In particular, fluorine-modified silicones, perfluoroalkylbiphenyl-modified silicones, and perfluoropolyethers are desirable because of their versatility.

Exmples of the moisturizing agent include sugar alcohols such as sorbitol, maltose, and maltitol; sterols such as cholesterol, sitosterol, phytosterol, and lanosterol; glucose, sucrose, lactose, raffinose, trehalose, xylitol, glycerin, propylene glycol, dipropylene glycol, tripropylene glycol, polypropylene glycol, 1,3-butylene glycol, ethylene glycol, diethylene glycol, triethylene glycol, polyglycerin, hyaluronic acid and its salts, chondroitin sulfuric acid and its salts, pyrrolidone carboxylic acid salts, polyoxyethylene methylglucoside, polyoxypropylene methylglucoside, and ethylglucoside.

The following compounds are used as the thickener: plant-derived polymers such as gum Arabic, tragacanth gum, arabinogalactan, locust bean gum (carob gum), guar gum, karaya gum, carrageenan, pectin, agar-agar, quince seed (i.e., marmelo), starch from rice, corn, potato or wheat, algae colloid, and trant gum; bacteria-derived polymers such as xanthan gum, dextran, succinoglucan, and pullulan; animal-derived polymers such as collagen, casein, albumin, and gelatin; starch-derived polymers such as carboxymethyl starch and methylhydroxypropyl starch; cellulose polymers such as methyl cellulose, ethyl cellulose, methylhydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose, and cellulose powder; alginic acid-derived polymers such as sodium alginate and propylene glycol alginate; vinyl polymers such as polyvinyl methylether, polyvinylpyrrolidone, and carboxyvinyl polymer; polyoxyethylene polymers such as polyethylene glycol; polyoxyethylene/polyoxypropylene copolymers; acrylic polymers such as sodium polyacrylate, polyethyl acrylate, and polyacrylamide; polyethyleneimine; cationic polymers; and inorganic thickening agents such as, bentonite, aluminum magnesium silicate, laponite, smectite, saponite, hectorite, and silicic anhydride.

An oil-soluble gelling agent may also be used as the thickening agent. For example, at least one may be selected from the following group: metal soaps such as aluminum stearate, magnesium stearate, and zinc myristate; α amino acid derivatives such as N-lauroyl-L-glutamic acid, α, γ-di-n-butylamine; dextrin fatty acid esters such as dextrin palmitate, dextrin stearate, and dextrin 2-ethylhexane palmitate; sucrose fatty acid esters such as sucrose palmitate and sucrose stearate; benzylidene derivatives of sorbitol such as monobenzylidene sorbitol and dibenzylidene sorbitol; clay minerals modified with an organic moiety such as dimethylbenzyldodecylammonium montmorillonite clay, dimethyldioctadecylammonium montmorillonite, and octadecyldimethylbenzylammonium montmorillonite.

As surfactants, other than the aforesaid nonionic surfactants, anionic surfactants and cationic surfactants can be used according to a cosmetic form. Examples of the anionic surfactants include fatty acid soaps, such as sodium stearate and triethanolamine palmitate, alkylether carboxylic acids and salts thereof, carboxylates of condensates from amino acids and fatty acids, alkyl sulfonic acids, alkenesulfonates, fatty acid ester sulfonates, fatty acid amide sulfonates, sulfonate salts of the formalin condensates with alkyl sulfonates, salts of sulfate esters such as salts of alkyl sulfates, salts of secondary higher alcohol sulfates, salts of alkyl/allyl ether sulfates, salts of fatty acid ester sulfates, salts of fatty acid alkylolamide sulfates, and Turkey Red oil, alkyl phosphates, ether phosphates, alkylallylether phosphates, amide phosphates, and N-acylamino surfactants. Examples of the cationic surfactants including amine salts such as alkylamine salts, polyamine and amino alcohol fatty acid derivatives, alkyl quaternary ammonium salts, aromatic quaternary ammonium salts, pyridium salts and imidazolium salts.

Examples of antiseptics, alkyl paraoxybenzoates, benzoic acid, sodium benzoate, sorbic acid, potassium sorbate, and phenoxyethanol may be used. For the antibacterial agents, benzoic acid, salicylic acid, carbolic acid, sorbic acid, paraoxybenzoic acid alkyl esters, parachloromethacresol, hexachlorophene, benzalkonium chloride, chlorohexydine chloride, trichlorocarbanilide, triclosan, photosensitizer and phenoxyethanol.

The bioactive components used in the present invention include materials which impart certain bioactivities to the skin when applied on the skin. For example, the following agents are used: anti-inflammatory agents, anti-aging agents, UV protection agents, astringents, antioxidants, hair growth stimulants, hair restoration tonics, humectants, blood circulation promoters, antibacterial agents, drying agents, cooling agents, hot poultice agent, vitamins, amino acids, wound healing promoters, anti-irritants, painkillers, cellular activators, and enzyme components. In particular, plant extracts, seaweed extracts, and herbal components from natural sources are desirable. In the present invention, one or more kinds of these bioactive components may preferably be added.

Examples of these components are as follows: Ashitaba extract, avocado extract, hydrangea extract, Althea extract, Arnica extract, aloe extract, apricot extract, apricot kernel extract, Ginkgo Biloba extract, fennel extract, turmeric[Curcuma] extract, oolong tea extract, rose fruit extract, Echinacea extract, Scutellaria root extract, Phellodendro bark extract, Japanese Coptis extract, Barley extract, Hyperium extract, White Nettle extract, Watercress extract, Orange extract, Dehydrated saltwater, seaweed extract, hydrolyzed elastin, hydrolyzed wheat powder, hydrolyzed silk, Chamomile extract, Carrot extract, Artemisia extract, Glycyrrhiza extract, hibiscus tea extract, Pyracantha Fortuneana Fruit extract, Kiwi extract, Cinchona extract, cucumber extract, guanocine, Gardenia extract, Sasa Albo-marginata extract, Sophora root extract, Walnut extract, Grapefruit extract, Clematis extract, Chlorella extract, mulberry extract, Gentiana extract, black tea extract, yeast extract, burdock extract, rice bran ferment extract, rice germ oil, comfrey extract, collagen, cowberry extract, Gardenia extract, Asiasarum Root extract, Family of Bupleurum extract, umbilical cord extract, Salvia extract, Saponaria extract, Bamboo extract, Crataegus fruit extract, Zanthoxylum fruit extract, shiitake extract, Rehmannia root extract, gromwell extract, Perilla extract, linden extract, Filipendula extract, peony extract, Calamus Root extract, white birch extract, Horsetail extract, Hedera Helix(Ivy) extract, hawthorn extract, Sambucus nigra extract, Achillea millefolium extract, Mentha piperita extract, sage extract, mallow extract, Cnidium officinale Root extract, Japanese green gentian extract, soybean extract, jujube extract, thyme extract, tea extract, clove extract, Gramineae imperata cyrillo extract, Citrus unshiu peel extract, Japanese Angellica Root extract, Calendula extract, Peach Kernel extract, Bitter orange peel extract, Houttuyna cordata extract, tomato extract, natto extract, Ginseng extract, garlic extract, wild rose extract, hibiscus extract, Ophiopogon tuber extarct, Nelumbo nucifera extract, parsley extract, honey, hamamelis extract, Parietaria extract, Isodonis herba extract, bisabolol extract, Loquat extract, coltsfoot extract, butterbur extract, Porid cocos wolf extract, extract of butcher's broom, grape extract, propolis extract, luffa extract, safflower extract, peppermint extract, linden tree extract, Paeonia extract, hop extract, pine tree extract, horse chestnut extract, Mizu-bashou [*Lysichiton camtschatcese]* extract, Mukurossi peel extract, Melissa extract, peach extract, cornflower extract, eucalyptus extract, saxifrage extract, citron extract, coix extract, mugwort extract, lavender extract, apple extract, lettuce extract, lemon extract, Chinese milk vetch extract, rose extract, rosemary extract, Roman Chamomile extract, and royal jelly extract.

Further examples of the bioactive components include the following: biopolymers such as deoxyribonucleic acid, mucopolysaccharides, sodium hyarulonate, sodium chondroitin sulfate, collagen, elastin, chitin, chitosan, and hydrolyzed chorionic membrane; amino acids such as glycine, valine, leucine, isoleucine, serine, threonine, phenylalanine, arginine, lysine, aspatic acid, glutamic acid, cystine, cysteine, methionine, and tryptophan; hormones such as estradiol and ethenylestradiol; moisturizing components such as amino acids, sodium lactate, urea, sodium pyrrolidonecarboxylate, betaine, and whey; oily components such as sphingolipids, ceramide, cholesterol, cholesterol derivatives, and phospholipids;
anti-inflammatory agents such as ε-aminocapronic acid, glycyrrhizic acid, β-glycyrrhetic acid, lysozyme chloride, guaiazulene, hydrocortisone, arantoin, tranexamic acid, and azulene; vitamins such as vitamin A, 82, B6, C, D, E, calcium pantothenate, biotin, nicotinic amide, and vitamin C ester; active components such as arantoin, diisopropylamine dichloroacetate, and 4-aminomethylcyclohexanoic acid; antioxidants such as tocopherol, carotinoide, flavonoid, tannin, lignan, saponin, butylhydroxyanisole, dibutylhydroxytoluene, and phytin; cellular activators such as α-hydroxy acid, β-hydroxy acid; blood circulation promoters such as γ-ryzanol and vitamin E derivatives; wound healing promoters such as retinol and retinol derivatives; refrigerants such as cepharantine, Glycyrrhiza extract, cayenne pepper tincture, hinokitiol, iodized garlic extract, pyridoxine hydrochloride, dl-α-tocopherol, dl-α-tocopherol acetic acid, nicotinic acid, nicotinic acid derivatives, calcium pantothenate, D-pantothenyl alcohol, acetyl pantothenyl ethylether, biotin, arantoin, isopropylmethylphenol, estradiol, ethinyl estradiol, carpronium chloride, benzalkonium chloride, diphenhydramine hydrochloride, tacanal, camphor, salicylic acid, nonylic acid vanillylamide, nonanoic acid vanillylamide, pirocton olamin, glyceryl pentadecanoate, 1-menthol, and camphor; hair restorers such as mononitro guaiacol, resolcinol, γ-aminobutylic acid, benzethonium chloride, mexiletine hydrochloride, auxin, female hormones, cantharis tincture, cyclosporin, zinc pyrithione, hydrocortisone, minoxyzil, polyoxyethylenesorbitan monostearate, peppermint oil, and sasanishiki extract.

Examples of the pH regulator include lactic acid, citric acid, glycolic acid, succinic acid, oxalic acid, dl-malic acid, calcium carbonate, sodium hydrogen carbonate, and ammonium hydrogen carbonate. Examples of the chelating agent include alanine, sodium edetate, sodium polyphosphate, sodium methaphosphate, and phosphoric acid.

Examples of the solvent to be used include light isoparaffin, ethers, LPG, N-methylpyrrolidone, and next-generation Flon besides purified water and mineral water.

In addition to the aforesaid cosmetic, the cosmetic containing M4Q may be skin care products, hair care products, makeup products, UV-ray protection products, and perfume products, for example, basic cosmetics such as milky lotions, creams, lotions, calamine lotions, sunscreen agents, sun tanning agents, aftershave lotions, preshave lotions, facial pack formulas, cleansing products, facial washes, acne remedy cosmetics, and essences; makeup cosmetics such as foundation, face powder, eye shadow, eyeliner, eyebrow, cheek, nail colors, lip cream, and lipstick; shampoos, rinses, conditioners, hair colors, hair tonics, hair-setting agents, body powder, hair restorers, deodorants, hair removers, soaps, body shampoos, bath agents, hand soaps, and perfume products. These may be in any form such as liquid form, milky liquid form, cream form, solid form, paste form, gel form, powder form, multiphase form, moose form, and spray form.

### EXAMPLES

The present invention will be explained in detail below by referring to Examples and Comparative Examples. However, the present invention shall not be limited to these examples. A content described below is expressed in mass% unless otherwise specified.

### Preparation Example

### (Preparation of Tetraquistrimethylsiloxysilane)

Tetraquistrimethylsiloxysilane was prepared according to the formulation shown in Tables 1 and 2 and the following processes 1 - 6.

Step 1: A 1000 ml four-neck glass flask was provided with a reflux condenser, a thermometer, and a stirrer and the air in the flask was purged with nitrogen gas. In this flask, hexamethyldisiloxane and alcohol were fed and then the flask was cooled in a ice water bath to make an interior temperature of 10 °C.

Step 2: To the flask, an acid catalyst was added dropwise while keeping the interior temperature at 10 °C and stirred for 30 minutes at that temperature.

Step 3: Then, alkoxysilane was added dropwise in 45 minutes while keeping the temperature at 10 °C and stirred for another 1 hour at that temperature.

Step 4: Water was added dropwise in one hour while keeping the interior temperature at 10 °C. After the addition completed, the reaction mixture was stirred for another one hour at a temperature of from 5 to 25°C.

Step 5: Heat was applied so as to raise the interior temperature to 50°C and then stirred for further 2 hours.

Step 6: The reaction mixture obtained was phase separated. After the aqueous layer was removed, the organic layer was washed with an aqueous solution of sodium bicarbonate and then with water. The organic layer was analyzed with gas chromatography results of which are as shown in Tables 1 and 2.

### [Gas Chromatographic Analysis Conditions]

Gas chromatograph: GC14B, ex Shimadzu Corp.
Column packing: SE30, trade name, 3 % of liquid phase
Column: inner diameter of 5 mm, and length of 3 m
Detector : TCD
Injection temperature: 80°C
Oven temperature: kept at 80°C for 1 minute and then raised to 300 °C at a temperature rising rate of 15 °C/min.

The organic layer obtained was distilled. A fraction was isolated at a temperature of from 89.5 to 90.0 °C and 1.3 kPa to obtain tetraquistrimethylsiloxysilane with a purity of 99.5 % or higher.

### [Distillation Conditions]

A distillation column with an inner diameter of 2.5 cm and a length of 70 cm was packed with McMahon packing material. Fractions of an objective substance and those of impurities were identified with mass spectrometry and NMR.

**Table 1**

| | Raw materials | Preparation Example 1 | Preparation Example 2 | Preparation Example 3 | Preparation Example 4 |
|---|---|---|---|---|---|
| Step 1 | Hexamethyldisiloxane | 486.0g | 648.0 | 486.0g | 486.0g |
| | | (3.0 moles) | (4.0 moles) | (3.0 moles) | (3.0 moles) |
| | Alcohol | Methanol 96.0g | Methanol 96.0g | None | Ethanol 46.0g |
| | | (3.0 moles) | (3.0 moles) | | (1.0 mole) |
| Step 2 | Acid catalyst | Concentrated sulfuric acid 9.8g | Concentrated sulfuric acid 9.8g | Concentrated sulfuric acid 9.8g | Trifluoromethane sulfonic acid 7.5g |
| | | (0.1 mole) | (0.1 mole) | (0.1 mole) | (0.05 mole) |
| Step 3 | Tetramethoxysilane | 152.0g | 152.0g | - | - |
| | | (1.0 mole) | (1.0 mole) | | |
| | Tetraethoxysilane | - | - | 208.0g | 208.0g |
| | | | | (1.0 mole) | (1.0 mole) |
| Step 4 | water | 90.0g | 90.0g | 90.0g | 72.0g |
| | | (5.0 moles) | (5.0 moles) | (5.0 moles) | (4.0 moles) |
| Analyses Results | | | | | |
| Results of midterm GC analysis of organic layer (area %) | M3Q(OMe) | 5.2 | 1.8 | - | - |
| | M3Q(OEt) | - | - | 4.5 | 6.7 |
| | M3Q(OH) | 1.1 | 0.3 | 0.9 | 1.8 |
| | Tetraquistrimethyl siloxysilane | 91.7 | 97.2 | 93.4 | 89.6 |
| | High boiling point fraction | 2.0 | 0.7 | 1.2 | 1.9 |
| Results of distillation | Yield (g) | 332.2 | 361.0 | 344.1 | 316.0 |
| | Yield | (%) 86.5 | 94.0 | 89.6 | 82.3 |
| | Pot yield (%) | 39.8 | 36.3 | 41.0 | 40.9 |
| Results of GC analysis of final pure product (area %) | M3Q(OMe) | less than 0.1 | less than 0.1 | - | - |
| | M3Q(OEt) | - | - | less than 0.1 | less than 0.1 |
| | M3Q(OH) | less than 0.1 | 0.3 | 0.2 | less than 0.1 |
| | Tetraquistrimethyl siloxysilane | 99.9 or more | 99.7 | 99.8 | 99.9 or more |
| | High boiling point fraction point | less than 0.1 | less than 0.1 | less than 0.1 | less than 0.1 |

**Table 2**

| | Raw materials | Preparation Example 5 | Preparation Example 6 |
|---|---|---|---|
| Step 1 | Hexamethyldisiloxane | 405.0g | 486.0 |
| | | (2.5 moles) | (3.0 moles) |
| | Alcohol | Methanol | Ethanol |
| | | 96.0g | 46.0g |
| | | (3.0 moles) | (1.0 mole) |
| Step 2 | Acid catalyst | Concentrated sulfuric acid | Concentrated sulfuric acid |
| | | 19.6g | 9.8g |
| | | (0.2 mole) | (0.1 mole) |
| Step 3 | Tetramethoxysilane | 152.0g | - |
| | | (1.0 mole) | |
| | Tetraethoxysilane | - | 152.0g |
| | | | (1.0 mole) |
| Step 4 | Water | 90.0g | 144.0g |
| | | (5.0 moles) | (8,0 moles) |
| Analyses Results | | | |
| Results of Results of midterm GC analysis of organic layer (area %) | M3Q(OMe) | 8.7 | - |
| | M3Q(OEt) | - | 4.4 |
| | M3Q(OH) | 3.1 | 0.6 |
| | Tetraquistrimethyl siloxysilane | 84.2 | 94.1 |
| | High boiling point fraction | 4.0 | 0.9 |
| Results of distillation Results of GC analyst of final pure product (area %) | Yield (g) | 309.1 | 346.4 |
| | Yield (%) | 80.5 | 90.2 |
| | Pot yield (%) | 40.5 | 41.3 |
| | M3Q(OMe) | less than 0.1 | - |
| | M3Q(OEt) | - | less than 0.1 |
| | M3Q(OH) | less than 0.1 | 0.2 |
| | Tetraquistrimethyl siloxysilane | 99.9 or more | 99.8 |
| | High boiling point fraction | less than 1 less than 0.1 | less 0.1 less than 0.1 |

In the tables, M3Q(OMe) represents ((CH₃)₃SiO)₃SiOCH₃, M3Q(OEt) represents {(CH₃)₃SiO)₃SiOCH₂CH₃, and M3Q(OH) represents {(CH₃)₃SiO}₃SiOH, i.e., 1,1,1,5,5,5-hexamethyl-3-trimethylsiloxy-3-hydroxytrisilo xane. The yield was determined as the molar ratio of the objective substance, M4Q, to the fed tetraalkoxysilane.

### Comparative Production Example

A hydrolysis reaction was carried out according to the formulation shown in Table 3 and the aforesaid steps 1 to 6. Distillation conditions were adjusted so as to obtain a fraction containing much M3Q(OH). To the fraction, purified tetraquistrimethylsiloxysilane was added so as to make a solution containing 35 % of M3Q(OH) and 65 % of tetraquistrimethylsiloxysilane. The results of GC analysis of the solution are shown in Table 4.

**Table 3**

| | Raw materials | Comparative Preparation Example |
|---|---|---|
| Step 1 | Hexamethyldisiloxane | 486.0 |
| | | (3.0 moles) |
| | Alcohol | Ethanol |
| | | 46.0g |
| | | (1.0 mole) |
| Step 2 | Acid catalyst | Concentrated sulfuric acid |
| | | 9.8g |
| | | (0.1 mole) |
| Step 3 | Tetramethoxysilane | - |
| | Tetraethoxysilane | 152.0g |
| | | (1.0 mole) |
| Step 4 | Water | 36.0g |
| | | (2.0 moles) |
| Analysis results | | |
| Results of midterm GC analysis of organic layer (area %) | M3Q(OMe) | - |
| | M3Q(OEt) | 0.7 |
| | M3Q(OH) | 18.2 |
| | M4Q | 65.8 |
| | High boiling point fraction | 15.3 |

**Table 4**

| | | |
|---|---|---|
| Results of GC Analysis (area %) | M3Q(OMe) | - |
| | M3Q(OEt) | |
| | M3Q(OH) | 35 |
| | M4Q | 65 |
| | High boiling point fraction | 0.1 |

Examples and Comparative Examples of the present cosmetic are shown below, wherein tetraquistrimethylsiloxysilane (M4Q) used was the one produced in Preparation Example 1. Methyltrimethicone used was TMF-1.5 produced by Shin-Etsu Chemical Co., Ltd.

### Example 1: Cleansing Gel

A cleansing gel was prepared according to the formulation and preparaion method described below.

| (Components) | (%) |
|---|---|
| 1. Ethanol | 3.0 |
| 2. Glycerol | 5.0 |
| 3. Dimethicone (Note 1) | 8.0 |
| 4. Tetraquistrimethylsiloxysilane | 15.0 |
| 5. Polyether-modified polysiloxane (Note 2) | 7.0 |
| 6. P.O.E.(60 moles) hydrogenated castor oil | 1.0 |
| 7. Octyldodecyl myristate | 2.0 |
| 8. Vitamin E acetate | 0.1 |
| 9. Oleyl alcohol | 0.3 |
| 10. Alkyl-modified carboxyvinyl polymer | 0.5 |
| 11. Carboxyvinyl polymer | 0.2 |
| 12. Methylparaoxy benzoate | 0.3 |
| 13. Calcium hydroxide | 0.4 |
| 14. Phnoxyethanol | 0.2 |
| 15. Purified water | Balance |

| | |
|---|---|
| Note 1: Silicone KF-96-100cs (produced by Shin-Etsu Chemical Co., Ltd.) Note 2: Silicone KF-351 (produced by Shin-Etsu Chemical Co., Ltd.) | |

### (Preparation Method)

Oily components 1 - 9 were homogeneously mixed and dissolved. Then, Components 10 - 15 were uniformly dispersed to which the mixture of the oily components previously obtained was added. The mixture thus obtained was homogenized to form a uniform dispersion which was packed in a tube to make a product.

### Example 2 Cleansing Gel

A cleansing gel having the following formulation was prepared in the same manner as in Example 1.

| (Components) | (%) |
|---|---|
| 1. Ethanol | 3.0 |
| 2. Glycerol | 5.0 |
| 3. Dimethicone (Note 1) | 5.0 |
| 4. Tetraquistrimethylsiloxysilane | 10.0 |
| 5. Methyltrimethicone (M3T) | 8.0 |
| 6. Polyether-modified polysiloxane (Note 2) | 7.0 |
| 7. P.O.E.(60 moles) hydrogenated castor oil | 1.0 |
| 8. Octyldodecyl myristate | 2.0 |
| 9. Vitamin E acetate | 0.1 |
| 10. Oleyl alcohol | 0.3 |
| 11. Alkyl-modified carboxyvinyl polymer | 0.5 |
| 12. Carboxyvinyl polymer | 0.2 |
| 13. Methylparaoxy benzoate | 0.3 |
| 14. Calcium hydroxide | 0.4 |
| 15. Phnoxyethanol | 0.2 |
| 16. Purified water | Balance |

| | |
|---|---|
| Note 1: Silicone KF-96-100cs (produced by Shin-Etsu Chemical Co., Ltd.) Note 2: Silicone KF-351(produced by Shin-Etsu Chemical Co., Ltd.) | |

### Comparative Example 1

Example 1 was repeated except that volatile cyclic silicone pentamer was used in place of tetraquistrimethylsiloxysilane.

### Comparative Example 2

Example 1 was repeated except that Silicone KF-96-100cs (produced by Shin-Etsu Chemical Co., Ltd.) was used in place of polyether-modified polysiloxane, and P.O.E.(60 moles) hydrogenated castor oil.

Sensory properties of each aforesaid cleansing agent were rated according to the criteria shown below.

### [Rating Criteria]

Each cleansing agent was evaluated by dedicated 20 panelists some of whom evaluated a plurality of agnets. Each cleansing agent was rated according to the number of the panelist who judged the agent good in each evaluation item as shown in the following table.

| The number of panelists out of 20 panelists who judged the agent good | Rating |
|---|---|
| 15 or more | A |
| 10 - 14 | B |
| 5 - 9 | C |
| 0 - 4 | D |

The results are as shown below.

### (Sensory Properties)

| | No dry feel after use | Thorough removal of makeup | Excellent usability |
|---|---|---|---|
| Example 1 | A | A | A |
| Example 2 | A | A | A |
| Comparative Example 1 | C | A | A |
| Comparative Example 2 | A | D | D |

From the results shown in the table above, the present cosmetics of Examples removed makeup better than those of Comparative Examples. They did not cause dry feel to have a better usability.

### Example 3 Dry Shampoo

A dry shampoo was prepared according to the formulation and the preparation method described below.

| (Components) | (%) |
|---|---|
| 1. Tetraquistrimethylsiloxysilane | 47 |
| 2. Methyltrimethicone | balance |
| 3. Ethanol | 12 |
| 4. Methylphenylpolysiloxane (KF 56, ex Shin-Etsu Chemical Co., Ltd.) | 8 |
| 5. Nonyl isononanate | 2 |
| 6. Menthol | 0.1 |
| 7. Perfume | q.s. |
| 8. Hibiscus extract | 0.2 |
| 9. Aloe extract | 0.1 |

### (Preparation Method)

Each component was homogeneously dissolved and packed in a container.

### Comparative Example 3

Example 3 was repeated except that volatile cyclic silicone pentamer was used in place of tetraquistrimethylsiloxysilane and methyltrimethicone.

### Example 4 Sheet Form Cleansing Agent

A sheet form cleansing agent was prepared according to the formulation and the preparation method described below.

| (Components) | (%) |
|---|---|
| 1. Tetraquistrimethylsiloxysilane | balance |
| 2. Ethanol | 20 |
| 3. Methylphenylpolysiloxane (KF 56, ex Shin-Etsu Chemical Co., Ltd.) | 4 |
| 4. 1,3-Butylene glycol | 10 |
| 5. Menthol | 0.1 |
| 6. Perfume | q.s. |

### (Preparation Method)

Each component was homogeneously dissolved. The solution thus obtained was poured with a dispenser on a sheet of nonwoven fabric which was enclosed in a pouch sealed container.

### Example 5. Sheet Form Cleansing Preparation

A sheet form cleansing agent was prepared according to the following formulation in the same manner as in Example 4.

| (Components) | (%) |
|---|---|
| 1. Tetraquistrimethylsiloxysilane | balance |
| 2. Methyltrimethicone (M3T) | 25 |
| 3. Ethanol | 15 |
| 4. Methylphenylpolysiloxane (KF 56, ex Shin-Etsu Chemical Co., Ltd.) | 4 |
| 5. 1,3-Butylene glycol | 10 |
| 6. Menthol | 0.1 |
| 7. Perfume | q.s. |

### Comparative Example 4

Example 4 was repeated except that a volatile cyclic silicone tetramer was used in place of tetraquistrimethylsiloxysilane.

Each cleansing preparation was evaluated according to the aforesaid rating criteria for sensory properties. The results are as shown below.

### (Sensory Properties)

| | No dry feel after use | Thorough removal of dirt | Excellent usability |
|---|---|---|---|
| Example 3 | A | B | B |
| Comparative Example 3 | D | C | C |
| Example 4 | A | A | A |
| Example 5 | A | A | A |
| Comparative Example 4 | D | C | C |

From the results shown in the above table, the present cleansing preparations of Examples removed dirt better than those of Comparative Examples. Further, they do not give dry feel to have superior usability.

The following table shows formulation of the perfume used in Examples 3 - 5.

### Fragrance formulation

| ingredient | mass 0/00 | ingredient | mass ‰ |
|---|---|---|---|
| terpineol | 10.00 | vanillin | 2.00 |
| terpinylacetate | 2.00 | ethyl vanillin | 0.10 |
| epi-methyl dihydorojasmonate | 60.00 | muscone | 0.50 |
| methyl dihydroasmonate | 250.00 | ethylene brassylate | 42.00 |
| indole | 0.05 | 4,6,6,7,8,8-hexamethyl-1,3,4,6,7,8-hexahydrocyclopentabenzopyran | 60.00 |
| 2-methyl-3-(3,4-methylenedioxy-phenyl)-propanal | 3.00 | cyclopentadecanolide | 20.00 |
| hydroxy citronellal | 20.00 | ambrettolide | 1.00 |
| hydroxy citronellol | 10.00 | γ-undecalactone | 0.40 |
| p-t-butyl-α-metylhydroxycinnamic | 35.00 | γ-decalactone | 0.10 |
| 4-(4-hydroxy-4-methyl-pentyl)-3-cyclohexen-1- | 75.00 | 4-(4-hydroxyphenyl)-2-butanone | 0.50 |
| 3-methyl-5-phenylpentanol | 20.00 | musk ketone | 0.10 |
| phenylethylalcohol | 10.00 | skatole | 0.01 |
| α-ionone | 10.00 | cis-jasmone. | 0.05 |
| β-ionone | 20.00 | phenylethylacetate | 0.10 |
| γ-mthyl ionone | 10.00 | civetone | 0.20 |
| dihydro-β-ionone | 25.00 | γ-nonalactone | 0.05 |
| benzylsalicylate | 150.00 | α-santalol | 0.20 |
| cis-3-hexenylsalicylate | 30.00 | β-santalol | 0.20 |
| eugenol | 0.80 | eugenylacetate | 0.10 |
| cinnamic alcohol | 5.00 | α-hexylcinnamic aldehyde | 20.00 |
| cinnamic aldehyde | 0.50 | α-damascone | 0.04 |
| guaiolacetate | 1.00 | β-damascone | 0.02 |
| guaiol | 0.50 | β-damascenone | 0.01 |
| cedrenylacetate | 5.00 | σ-damascone | 0.01 |
| cedrylmethylketone | 30.00 | rose absolute | 0.50 |
| 6,7-dihydro-1,1,2,3,3-pentamethyl-4(5H)-indan | 2.00 | rose oil | 4.50 |
| Vetiveracetate | 10.00 | sandal wood oil | 2.00 |
| 3-methyl-5-(2,3,3-trimethyl-3-cyclopentene-1-il)-pentane-2-ol | 2.00 | labdanum absolute | 0.05 |
| 3-ethyl-4-(2,3,3-trimethyl-3-cyclopentene-1-il)-2-butene-1-ol | 0.80 | ciste absolute | 0.01 |
| isobornylcyclohexanol | 35.00 | Vetiver oil | 0.05 |
| heliotropin | 10.00 | guaiac wood oil | 0.10 |
| coumarin | 2.00 | Total | 1000.00 |

### Example 6. Lipstick

A lipstick was prepared according to the formulation and preparation method described below. As coloring pigments, pigements treated with 8 mass % of Nε-lauloyl-L-lysine were used. Titanated mica was treated with 10 mass % of Nε-lauloyl-L-lysine. Titanium oxide was treated with 8 mass % of octylsilane.

### (Preparation of dispersion liquid of color pigment)

With 30 parts by weight of surface treated Red No.201, 70 parts by weight of tetraquistrimethylsiloxysislane was mixed. Dispersion liquid A was prepared by passing the pigment mixture and medium through a sand mill two times.

With 30 parts by weight of surface treated Red No.202, 70 parts by weight of tetraquistrimethylsiloxysislane was mixed. Dispersion liquid B was prepared by passing the pigment mixture and a medium through a sand mill two times.

| (Component A) | |
|---|---|
| 1. Dipersion liquid A | 3 |
| 2. Dispersion liquid B | 8 |
| 3. Tetraquistrimethylsiloxysilane | 30 |
| (Component B) | |
| 4. Ceresin | 18 |
| 5. Cator oil | balance |
| 6. Polybutene | 8 |
| 7. Alkyl-modified silicone | 1 |
| (Component C) | |
| 8. Treated titanium oxide | 1 |
| (Component D) | |
| 9. Treated titanated mica | 14 |

### (Preparation Method)

Component B was prepared by mixing and dissolving, with which Component A was mixed. In the mixture obtained, Component C was dispersed and kneaded, to which Component D was added and dispersed uniformly. The mixture obtained was degassed and poured in a metal mold followed by cooling to solidify into a product.

### Comparative Example 5

Example 6 was repeated except that a cyclic silicone pentamer(decamethylcyclopentasiloxane) was used in place of tetraquistrimethylsiloxysilane .

### Comparative Example 6

Example 6 was repeated except that diperson liquids prepared only by mixing without pulvelization by the sand mill were used in place of the dipersion liquid A and B, respectively, and the following preparaion method was used instead of the aforesaid method.

### (Preparation Method)

Component B was prepared by mixing and dissolving and mixed with Component C. After the mixture was kneaded, Component , A and Component D were dispersed. The mixture was degassed and then poured in a metal mold and then cooled to solidify into a product.

In the following table, evaluation results of sensory properties of Example 6, Comparative Examples 5 and 6 are shown.

### (Sensory Properties)

| | Brightness of color | Translucency of color | Smoothness in application | Long-lasting color |
|---|---|---|---|---|
| Example 6 | A | A | A | B |
| Comparative Example 5 | C | C | B | B |
| Comparative Example 6 | D | D | D | D |

Lipsticks of Examples were found to have brighter color and higher translucency; they were applied smoothly on the lip and the applied film showed no or little color fade. In contrast, the lipstick of Comparative Example 5 containing cyclic silicone, which is a conventionally used dispersion medium, showed inferior color tone due to poorer dispersion. The lipstick of Comparative Example 6 was prepared without dispersing process and unusable as a lipstick.

### Example 7. Lipstick

A lipstick was prepared according to the formulation and preparation method described below. As coloring pigments, pigements treated with 5 mass % of Nε-lauloyl-L-lysine were used.

| (Component A) | |
|---|---|
| 1. Tetraquistrimethylsiloxysilane | 40 |
| 2. Trimethylsiloxysilicate | 2 |

| (Component B) | |
|---|---|
| 3. Ceresin | 18 |
| 4. Cator oil | balance |
| 5. Polybutene | 8 |

| (Component C) | |
|---|---|
| 6. Treated Red No.202 | 1 |
| 7. Treated titanium oxide | 1 |
| 8. Treated titanated mica | 15 |

Componentn B was prepared by mixing and dissolving, in which Component C was dispersed. The dispersion was kneaded, to which Component A which had been premixed to a solution. The mixture obtained was degassed and poured in a metal mold to solidify into a lipstick.

### Example 8 Lipstick

A lipstick having the following formulation was prepared as in Example 7.

| | |
|---|---|
| 1. Tetraquistrimethylsiloxysilane | 20 |
| 2. Mehtyltrimethicone(M3T) | 20 |
| 3. Trimethylsiloxysilicate | 3 |
| 4. Ceresin | 8 |
| 5. Cator oil | 17 |
| 6. Polybutene | 10 |
| 7. Methylphenylpolysiloxane(EZ-209: ex Nihon Nippon Unicar Company Limited | 5 |
| 8. Treated Red No.202 | 1 |
| 9. Treated titanium oxide | 1 |
| 10. Treated titanated mica | 15 |

### Comparative Example 7

Example 7 was repeated except that cyclic silicone pentamer(decamethylcyclopentasiloxane) was used in place of tetraquistrimethylsiloxysilane.

### Comparative Example 8

Example 7 was repeated except that ceresin was used in place of polybutene.

### Comparative Example 9

Example 7 was repeated except that dimethylpolysiloxane(KF 96A having a viscosity of 100 cs, ex Shin-Etsu Chemial Co., Ltd., was used in place of tetraquistrimethylsiloxysilane.

Results of sensory evaluation of Examples 7, 8 and Comparative Examples 7 - 9 are as shown in the table below.

### (Sensory Properties)

| | Long-lasting | gloss | Bright color | No dry feel. | Smoothness in application | Good spreadability |
|---|---|---|---|---|---|---|
| Example 7 | A | A | A | A | A | A |
| Example 8 | A | A | A | A | A | A |
| Comp. Ex. *7 | B | C | C | D | B | B |
| Comp. Ex. 8 | D | D | D | D | D | D |
| Comp. Ex. 9 | D | B | C | C | D | C |

| | | | | | | |
|---|---|---|---|---|---|---|
| * Comparative Example | | | | | | |

From the above results, lipsticks of Examples were superior to those of Comparative Examples in each evaluation item. The lipstick containing the conventionally used cyclic silicone instead of tetraquistrimethylsiloxysilane gave dry feel and showed inferior brightness of the color. The lipstick of Comparative Example 8 containing ceresin instead of polybutene was rated low. The lipstick of Comparative Example 9 contained no volatile silicone but non-volatile silicone, which lipstick was rated low as a whole.

### Example 9: Foundation

A solid foundation was prepared according to the formulation and the preparation method described below. As coloring pigments, pigements treated with 5 mass % of Nε-lauloyl-L-lysine were used.

### [Pigement Portion] (38.2 mass%)

| | |
|---|---|
| 1. Treated spherical titanium oxide(average primary particle size of 0.4 µm) | 18 |
| 2. Treated iron oxide( a mixture of iron oxide black, iron oxide red, and iron oxide yellow) | 1.7 |
| 3. Treated talc | 6 |
| 4. Treated mica | 2 |
| 5. Nε-lauloyl-L-lysine | 3.5 |
| 6. Polyalkylmethylsesquioxane(average primary particle size of 4 µm) | 5 |
| 7. Octylsililated titanium oxide fine powder [Liquid Portion] Volatile silicone (25 mass%) | 2 |
| 8. Tetraquistrimethylsiloxysilane Polyol (6.5 mass%) | 25 |
| 9. 1,3-butyleneglycol | 5 |
| 10. Maltitol | 1 |
| 11. Raffinose Surfactant (2 mass%) | 0.5 |
| 12. Sorbitan isostearate Solid or pasty oil agent(5 mass%) | 2 |
| 13. Paraffin Purified water (12.7 mass%) | 5 |
| 14. Purified water Oil agents | 12.7 |
| 15. Dimethylpolysiloxane (6cS) | 3 |
| 16. Methyphenylpolysiloxane | 2 |
| 17. Octyl paramethoxycinnamate | 2 |
| 18. Propylene glycol dicaprilate | 2 |
| 19. Dipentaerythrityl hexahydroxystearate Bioactive component | 0.5 |
| 20. Cranberry extract Antiseptic | 1 |
| 21. Paraoxybenzoate | 0.1 |

### (Preparation Method)

Liquid oil agents were uniformly dissolved by mixing at 80 °C, to which aqueous liquid portion which had been uniformly dissolved by mixing at 80 °C was added and emulsified. The emulsion thus obtained was degassed and packed in a metal plate. The plate was placed in a sealable container to obtained solid foundation.

### Example 10: Foundation

A solid foundation having the following formulation was prepared as in Example 9.

### [Pigement Portion] (38.2 mass%)

| | |
|---|---|
| 1. Treated spherical titanium oxide(average primary particle size of 0.4 µm) | 18 |
| 2. Treated iron oxide (a mixture of iron oxide black, iron oxide red, and iron oxide yellow) | 1.7 |
| 3. Treated talc | 6 |
| 4. Treated mica | 2 |
| 5. Nε-lauloyl-L-lysine | 3.5 |
| 6. Polyalkylmethylsesquioxane(average primary particle size of 4 µm) | 5 |
| 7. Octylsililated titanium oxide fine powder [Liquid Portion] Volatile silicone (25 mass%) | 2 |
| 8. Tetraquistrimethylsiloxysilane | 15 |
| 9. Methyltrimethicone Polyol (6.5 mass%) | 10 |
| 10. 1,3-butyleneglycol | 5 |
| 11. Maltitol | 1 |
| 12. Raffinose Surfactant (2 mass%) | 0.5 |
| 13. Sorbitan isostearate Solid or pasty oil agent(5 mass%) | 2 |
| 14. Paraffin Purified water (12.7 mass%) | 5 |
| 15. Purified water Oil agents | 12.7 |
| 16. Dimethylpolysiloxane (6cS) | 3 |
| 17. Methyphenylpolysiloxane | 2 |
| 18. Octyl paramethoxycinnamate | 2 |
| 19. Propylene glycol dicaprilate | 2 |
| 20. Dipentaerythrityl hexahydroxystearate Bai active component | 0.5 |
| 20. Cranberry extract Antiseptic | 1 |
| 21. Paraoxybenzoate | 0.1 |

### Comparative Example 10

Example 9 was repeated except that volatile cyclic silicone pentamer was used in place of tetraquistrimethylsiloxysilane.

### Comparative Example 11

Example 9 was repeated except that tetraquistrimethylsiloxysilane was used in place of purified water (37.7 mass% of tetraquistrimethylsiloxysilane).

### Comparative Example 12

Example 9 was repeated except that methylpolysiloxane(10 cS) was used in place of tetraquistrimethylsiloxysilane.

### Comparative Example 13

Example 9 was repeated except that liquid paraffin was used in place of polyol.

### Comparative Example 14

Example 9 was repeated except that liquid paraffin was used in place of the paraffin used in Example 9.

### Comparative Example 15

Example 9 was repeated except that a content of the paraffin was increased to 10 mass % which was compensated by a decreased amount of water.

### Comparative Example 16

Example 9 was repeated except that a conent of sorbitan isostearate was reduced to 1 mass % which was compensated by an increased amount of water.

### Comparative Example 17

Example 9 was repeated except that a content of sorbitan isostearate was increased to 5 mass % which was compensated by a decreased amount of purified water.

### Comparative Example 18

Example 9 was repeated except that a content of water was decreased to 0 mass % which was compensated by an increased amount of 1,3-butylene glycol.

### Comparative Example 19

Example 9 was repeated except that purified water was used in place of tetraquistrimethylsiloxysilane.

### Comparative Example 20

Example 9 was repeated except that a content of each pigment was increased by 1.3 times (a total content of the pigments: 9.66 mass%), and a content of tetraquistrimethylsiloxysilane was decreased to 13.54 mass%.

### Comparative Example 21

Example 9 was repeated except that a content of each pigment was decreased to one-fourth of that in Example 9 (a total content of the pigments: 9.55 mass%), and a content of tetraquistrimethylsiloxysilane was increased to 53.65 mass%.

Each foundation was evaluated as described below.

### [Evaluation of Effects on the Skin]

Each foundation was evaluated by dedicated 10 panelists, some of whom evaluated a plurality of foundation, and was scored according to the criteria shown below. The ratings of all the panelists were totaled. A higher score means that a higher effect was observed (Full score: 50 points).

### Scoring criteria in evaluation of effects on the skin

| Criteria | Point |
|---|---|
| Significant effect was appreciated | 5 |
| Fair effect was appreciated | 4 |
| Effect was appreciated | 3 |
| A little effect was appreciated | 2 |
| No effect was appreciated | 1 |

### Evaluation results are as shown below.

| | Excellent affinity for the skin | Comfortable to apply | Long | No dry feel when applied | Non-greasy |
|---|---|---|---|---|---|
| Example 9 | A | A | A | A | A |
| Example 10 | A | A | A | A | A |
| Comparative Example 10 | A | B | A | D | B |
| Comparative Example 11 | C | C | B | A | D |
| Comparative Example 12 | D | D | D | A | D |
| Comparative Example 13 | D | D | C | A | D |
| Comparative Example 14 | D | B | C | A | B |
| Comparative Example 15 | D | D | D | A | B |
| Comparative Example 16 | B | C | C | A | C |
| Comparative Example 17 | C | C | C | A | D |
| Comparative Example 18 | D | D | D | D | C |
| Comparative Example 19 | D | D | B | A | B |
| Comparative Example 20 | D | D | C | C | D |
| Comparative Example 21 | B | B | B | B | C |

Stability of each foundation was evaluated according to the following criteria.

### [Evaluation of Stability]

Samples from each Example and Comparative Example were prepared according to a predetermined method and kept at 0°C, 25°C, and 45°C, respectively. Samples were visually observed whether there is separation or agglomeration at any one of the temperatures and rated according to the following critera.

| Criteria | Rating |
|---|---|
| No separation or agglomeration | A |
| A little separation or agglomeration | B |
| Separation and agglomeration | C |

The results are as shown below.

### [Stability of cosmetic]

| | Stability |
|---|---|
| Example 9 | A |
| Example 10 | A |
| Comparative Example 10 | A |
| Comparative Example 11 | C |
| Comparative Example 12 | B |
| Comparative Example 13 | B |
| Comparative Example 14 | C |
| Comparative Example 15 | A |
| Comparative Example 16 | B |
| Comparative Example 17 | C |
| Comparative Example 18 | C |
| Comparative Example 19 | C |
| Comparative Example 20 | C |
| Comparative Example 21 | C |

From the results shown in the above table, Examples of the present invention are superior in affinity for the skin, feel to the touch, water resistance, sebum resistance, and stability than Comparative Examples. Foundation of Comparative Example 10 contained cyclic silicone pentamer to be inferior to Examples in greasiness and dry feel.

Foundation of Comparative Example 11 contained a lot of M4Q to be greasy, resulting in lower rating.

Foundation of Comparative Example 12 did not contain volatile solvent. It was highly greasy to give bad feel to the touch with tackiness.

Foundation of Comparative Example 14 did not contain solid oil. It had so low viscosity that it spilled from a container.

Foundation of Comparative Example 15 contained a lot of solid oil. It was hard to form a cake.

Foundation of Comparative Example 16 contained a smaller amount of surfactant to be less stable.

Foundation of Comparative Example 17 contained a lot of surfactant to fail to have good feel to the touch.

Foundation of Comparativ Example 18 contained no water to be inferior in the stability and the feel to the touch.

Foundation of Comparative Example 19 contained a lot of water to be inferior in the feel, cuasing strained feel.

Foundation of Comparative Example 20 contained larger amount of pigment. It was inferior in the feel and the stability due to the unbalanced formulation.

Foundation of Comparative Example 21 contained less amount of pigment. It's feel was good enough but the stability was bad due to the unbalanced formulation. Further, it was difficult to take out from a container.

### Example 11: UV-ray Protective Cosmetic Base

A solution of trimethylsiloxysilicate, a kind of silicone resin, dissolved in tetraquistrimethylsiloxysilane at a concentration of 50% by mass was prepared and a UV-ray protective cosmetic base was prepared according to the formulation shown in the table below.

| (Component A) | % |
|---|---|
| (1) Silicone-treated titanium dioxide fine particle | 4 |
| (2) Methyltrimethicone | 10 |
| (3) tetraquistrimethylsiloxysilane | 15 |
| (4) Polyether-modified silicone(KF6017, ex Shin-Etsu Chemical Co., Ltd.) | 1 |
| (Component B) | |
| (5) Silicone-treated zinc oxide fine particle | 6 |
| (6) Perfluoroalkylphosphate-treated colored skin-color mica | 0.5 |
| (Component C) | |
| (7) Crosslinked organopolysiloxane spherical powder (Elastomer) | 4 |
| (8) Dimethylpolysiloxane (KF96A-6) | 2 |
| (9) Fluorinated dimethiconol | 1 |
| (10) Trimethylsiloxysilicate solution | 6 |
| (11) Octyl paramethoxycinnamate | 3 |
| (12) Perfluoropolyether | 0.5 |
| (Component D) | |
| (13) Ethyl alcohol | 10 |
| (14) Purified water | Balance |
| (15) Aloe extract | 1 |
| (16) Cranberry extract | 1 |
| (17) Hibiscus extract | 0.5 |

| | |
|---|---|
| KF6017 (produced by Shin-Etsu Chemical Co., Ltd.): Polyoxyethylene/methylpolysiloxane copolymer (HLB = 4.6) KF96A-6 (produced by Shin-Etsu Chemical Co., Ltd.): Dimethylpolysiloxane with viscosity of 6 mm²/s | |

### (Preparation Method)

Step 1: Component A was ground with a roller mill to form a paste.
Step 2: Component C was roughly mixed and ground well with a mixer.
Step 3: After mixing Component B with Component C and forming dispersion, Component A was added thereto and mixed well.
Step 4: Component D which has been made into a homogeneous solution was added and stirred and then the mixture obtained was packed together with a stainless ball in a container to obtain a product.

### Comparative Example 22

Example 11 was repeated except that volatile cyclic silicone hexamer was used in place of tetraquistrimethylsiloxysilane.

### Example 12

Example 11 was repeated except that tetraquistrimethylsiloxysilane was used in place of methyltrimethicone.

### Comparative Example 23

Example 11 was repeated except that methyltrimethicone was used in place of tetraquistrimethylsiloxysilane. Examples 13, 14, and 15: Whitening Cream for Daytime Use A whitening cream was prepared in the formulation shown below. A mixed solution of tetraquistrimethylsiloxysilane and methyltrimethicone in 1:1 ratio was used.

| Example No. | 13 | 14 | 15 |
|---|---|---|---|
| (Component A) (%) | | | |
| (1) KF6017 | 1 | 1 | 1 |
| (2) KF6026 | - | - | 2 |
| (3) KF56 | 5 | 5 | 5 |
| (4) KF995 | 12 | 3 | - |
| (5) The mixed solution | 10 | 19 | 12 |
| (Component B) | | | |
| (6) Glycerin | 5 | 5 | 5 |
| (7) Dipropylene glycol | 10 | 10 | 10 |
| (8) Methyl paraoxybenzoate | 0.2 | 0.2 | 0.2 |
| (9) Sodium ascorbyl sulfate | 0.1 | 0.1 | 0.1 |
| (10) Sodium ascorbyl phosphate | 0.1 | 0.1 | 0.1 |
| (11) γ- amino butyric acid | 0.1 | 0.1 | 0.1 |
| (12) Apple seed kernel extract (antioxidant) | 0.1 | 0.1 | 0.1 |
| (13) Sodium chloride | 0.9 | 0.9 | 0.9 |
| (14) Perfume | 0.1 | 0.1 | 0.1 |
| (15) Purified water | Balance | Balance | Balance |

| | | | |
|---|---|---|---|
| KF6017 (produced by Shin-Etsu Chemical Co., Ltd.): Polyoxyethylene/methylpolysiloxane copolymer (HLB = 4.6) KF6026 (produced by Shin-Etsu Chemical Co., Ltd.): Polyoxyethylenemethylsiloxane/polyoxypropyleneoleylmethyl siloxane/dimethylsiloxane copolymer (HLB = 4.7) KF56 (produced by Shin-Etsu Chemical Co., Ltd.): Methylphenylpolysiloxane KF995 (produced by Shin-Etsu Chemical Co. Ltd.): Decamethylcyclopentasiloxane (D5) | | | |

### (Preparation Method)

Step 1: Component A was dissolved by heating at 60°C.
Step 2: Component B was dissolved by heating at 60°C.
Step 3: Component A was added to Component B while stirring to form an emulsion.
Step 4: Subsequently, the mixture was cooled to 30°C while stirring and packed in a container to obtain a product.

### Comparative Example 24

Example 14 was repeated except that ethanol was used in place of the mixed solution.

### Comparative Example 25

Example 15 was repeated except that light liquid paraffin was used in place of the mixed solution.

### Comparative Example 26

Example 15 was repeated except that tetraquistrimethylsiloxysilane containing 35 % of 1,1,1,5,5,5-hexamethyl-3-trimethylsiloxy-3-hydroxytrisilo xane prepared in Comparative Preparation Example 1 was used in place of the mixed solution.

### Comparative Example 27

Example 15 was repeated except that tetraquistrimethylsiloxysilane containing 5 % of 1,1,1,5,5,5-hexamethyl-3-trimethylsiloxy-3-hydroxytrisilo xane prepared by mixing tetraquistrimethylsiloxysilane containing 35 % of 1,1,1,5,5,5-hexamethyl-3-trimethylsiloxy-3-hydroxytrisilo xane prepared in Comparative Preparation Example 1 and tetraquistrimethylsiloxysilane was used in place of the mixed solution.

### Example 16: Sunscreen Agent

A sunscreen agent was prepared according to the formulation described below. The UV-ray protective components used were 2-ethylhexyl paramethoxycinnamate, treated fine particle titanium dioxide, treated fine particle zinc oxide, and treated yellow fine particle titanium dioxide. As a silicone resin compound, trimethylsiloxysilicate was used.

The treated titanium dioxide fine particle used was prepared by coating titanium dioxide fine particle having a mean particle size of 17 nm coated with silica/alumina with 8 mass% octyltrimethoxysilane and heat-treating at 160°C. The treated zinc oxide fine particle was prepared by coating zinc oxide fine particle having a mean particle size of 50 nm treated with silica with 3 mass % of methylhydrogenpolysiloxane and heat treating at 170°C. The treated yellow titanium dioxide fine particle was prepared by coating iron-doped titanium dioxide fine particle treated with silica with 3 mass % of methylhydrogenpolysiloxane and heat treating at 130°C.

| (Component A) | (%) |
|---|---|
| Treated titanium dioxide fine particle | 8.0 |
| Tetraquistrimethylsiloxysilane | 9.0 |
| Methyltrimethicone | 3.0 |
| (Component B) | |
| Treated yellow titanium dioxide fine particle | 0.8 |
| Treated zinc oxide fine particle | 12.0 |
| (Component C) | |
| Three-dimensionally crosslinked organopolysiloxane spherical powder (Elastomer) | 1.0 |
| Dimethiconol | 6.0 |
| Tetraquistrimethylsiloxysilane | 15.0 |
| Trimethylsiloxysilicate | 6.0 |
| 2-ethylhexyl paramethoxycinnamate | 10.0 |
| (Component D) | |
| Ethyl alcohol | 13.0 |
| Purified water | Balance |
| Aloe extract | 0.5 |

Component A was ground with a roller mill to form a paste. Component C was roughly mixed and thoroughly ground with a mixer. After mixing Component B with Component C to make dispersion, Component A was added and the mixture was further mixed well. Subsequently, Component D, which had been dissolved homogeneously, was added and stirred thoroughly, and then the mixture was packed with a stainless ball in a container.

### Comparative Example 28

Example 16 was repeated except that volatile linear silicone tetramer was used in place of tetraquistrimethylsiloxysilane.

### Example 17

Example 16 was repeated except that tetraquistrimethylsiloxysilane was used in place of methyltrimethicone.

### Comparative Example 29

Example 16 was repeated except that methyltrimethicone was used in place of tetraquistrimethylsiloxysilane.

### Example 18: Sunscreen Agent (Cream)

| (Components) | (%) |
|---|---|
| 1. Methyltrimethicone | 3.0 |
| 2. Tetraquistrimethylsiloxysilane | 17.0 |
| 3. Liquid paraffin | 10.0 |
| 4. KF6017 | 1.9 |
| 5. KF6026 | 4.0 |
| 6. 4-t-butyl-4'-methoxydibenzoylmethane | 7.0 |
| 7. Distearyldimethylammonium chloride | 0.8 |
| 8. Vitamin E acetate | 0.1 |
| 9. Ethanol | 1.0 |
| 10. Sumectite | 1.2 |
| 11. Antiseptic | q.s. |
| 12. Perfume | q.s. |
| 13. Purified water | Balance |

| | |
|---|---|
| KF6017 (produced by Shin-Etsu Chemical Co., Ltd.): Polyoxyethylene/methylpolysiloxane copolymer (HLB = 4.6) KF6026 (produced by Shin-Etsu Chemical Co., Ltd.): Polyoxyethylenemethylsiloxane/polyoxypropyleneoleylmethyl siloxane/dimethylsiloxane copolymer (HLB = 4.7) | |

### (Preparation Method)

A: Components 1 through 8 and 11 were mixed while heating.
B: Components 9, 10 and 11 were heated and mixed to make homogeneous dispersion.
C: While stirring, the dispersion from B was added portionwise to the mixture from A to emulsify and, after cooling, Component 12 was added to obtain a sunscreen agent (cream).

### Example 19: Sunscreen Agent (Cream)

| (Components) | (%) |
|---|---|
| 1. Tetraquistrimethylsiloxysilane | 18.0 |
| 2. KF56 | 2.0 |
| 3. Liquid paraffin | 1.5 |
| 4. KF6012 | 4.0 |
| 5. Octyl paramethoxycinnamate | 5.0 |
| 6. 1,3-butylene glycol | 4.0 |
| 7. Sodium chloride | 1.0 |
| 8. Antiseptic | q.s. |
| 9. Perfume | q.s. |
| 10. Purified water | Balance |

| | |
|---|---|
| KF56 (produced by Shin-Etsu Chemical Co., Ltd.): Methylphenylpolysiloxane KF6012 (produced by Shin-Etsu Chemical Co., Ltd.): Polyoxyethylene/polyoxypropylene/methylpolysiloxane copolymer (HLB = 7.0) | |

### (Preparation Method)

A: Components 1 - 6 were mixed while heating.
B: Components 7 - 9 and 10 were heated to be dissolved.
C: While stirring, B was added portionwise to A to emulsify and, after cooling, Component 10 was added to obtain a sunscreen agent (cream).

### Example 20: Sunscreen Agent (Cream)

| (Components) | (%) |
|---|---|
| 1. Tetraquistrimethylsiloxysilane | 16.5 |
| 2. Methyltrimethicone | 1.0 |
| 3. KP545 | 12.0 |
| 4. Glycerol triisooctanoate | 5.0 |
| 5. Octyl paramethoxycinnamate | 6.0 |
| 6. KSG21 | 5.0 |
| 7. KF6017 | 1.0 |
| 8. Lipophilic-treated zinc oxide | 20.0 |
| 9. Sodium chloride | 0.5 |
| 10. 1,3-butylene glycol | 2.0 |
| 11. Antiseptic | q.s. |
| 12. Perfume | q.s. |
| 13. Purified water | Balance |

| | |
|---|---|
| KP545 (produced by Shin-Etsu Chemical Co., Ltd.): Acrylic silicone copolymer resin / decamethylcyclopentasiloxane 30% solution KSG21 (produced by Shin-Etsu Chemical Co., Ltd.): Crosslinked polyether-modified methylpolysiloxane /dimethylpolysiloxane KF6017 (produced by Shin-Etsu Chemical Co., Ltd.): Polyoxyethylene/methylpolysiloxane copolymer (HLB = 4.6) | |

### (Preparation Method)

A: Component 3 was added to a portion of Component 1 to obtain a homogeneous mixture and then Component 8 was added to be dispersed with a beads mill.
B: The remaining portion of Component 1, Component 2 and Components 4 - 7 were mixed homogeneously.
C: Components 9 - 11 and Component 13 were mixed to dissolve. D: C was added to B to emulsify, and A and Component 12 were added to obtain a sun screen cream.

### Example 21: Sunscreen Agent (Astringent)

| (Components) | (%) |
|---|---|
| 1. Methyltrimethicone | 13.0 |
| 2. Tetraquistrimethylsiloxysilane | 1.0 |
| 3. KF615A | 10.0 |
| 4. Squalane | 1.5 |
| 5. Octyl paramethoxycinnamate | 3.0 |
| 6. Titanium TTO-S2 | 2.0 |
| 7. polymethylsilsesquioxane | 0.7 |
| 8. 1,3-butylene glycol | 10.0 |
| 9. Sodium chloride | 2.0 |
| 10. L-proline | 0.1 |
| 11. 2-hydroxyoctanoic acid | 1.0 |
| 12. 2-hydroxypropanpic acid | 5.0 |
| 13. Sodium hydroxide | q.s. |
| 14. Antiseptic | q.s. |
| 15. Perfume | q.s. |
| 16. Purified water | Balance |

| | |
|---|---|
| KF615A (produced by Shin-Etsu Chemical Co., Ltd.): Polyoxyethylene/polyoxypropylene/methylpolysiloxane copolymer (HLB = 14.0) Titanium TTO-S2 (produced by Ishihara Sangyou Co., Ltd.): Ultra-fine particle titanium dioxide treated for hydrophobicity | |

### (Preparation Method)

A: Components 8 - 16 were dissolved homogeneously.
B: Components 1 - 5 were mixed and Components 6 and 7 were added to obtain a homogeneous mixture.
C: While stirring, B was added portionwise to A to emulsify to obtain a sun blocking astringent.

### Example 22: Sunscreen Agent (Milky Lotion)

| (Components) | (%) |
|---|---|
| 1. Tetraquistrimethylsiloxysilane | 25.0 |
| 2. Diglyceryl monoisostearate | 1.5 |
| 3. Decaglyceryl pentaisostearate | 1.5 |
| 4. KF6012 | 0.5 |
| 5. Olive oil | 1.0 |
| 6. Titanium dioxide fine particle | 7.0 |
| 7. Glycerin | 5.0 |
| 8. Sodium chloride | 1.5 |
| 9. Antiseptic | q.s. |
| 10. Perfume | q.s. |
| 11. Purified water | Balance |

| | |
|---|---|
| KF6012 (produced by Shin-Etsu Chemical Co., Ltd.): Polyoxyethylene/polyoxypropylene/methylpolysiloxane copolymer (HLB = 7.0) | |

### (Preparation Method)

A: Components 1 - 5 were mixed while heating and Component 6 was dispersed homogeneously.
B: Components 7 - 9 and Component 11 were mixed while heating.
C: While stirring, B was added portionwise to A to emulsify and, after cooling, Component 10 was added to obtained a sunscreen agent (milky lotion).

### Example 23: Sunscreen Agent (Milky Lotion)

| (Components) | (%) |
|---|---|
| 1. Tetraquistrimethylsiloxysilane | 15.0 |
| 2. Ethanol | 5.0 |
| 3. KF56 | 3.0 |
| 4. Sorbitan monoisostearate | 1.0 |
| 5. KF6012 | 0.5 |
| 6. Silicone resin | 1.0 |
| 7. Octyl paramethoxycinnamate | 4.0 |
| 8. Titanium dioxide fine particle | 8.0 |
| 9. Sorbitol | 2.0 |
| 10. Sodium chloride | 2.0 |
| 11. Antiseptic | q.s. |
| 12. Perfume | q.s. |
| 13. Purified water | Balance |

| | |
|---|---|
| KF56 (produced by Shin-Etsu Chemical Co., Ltd.): Methylphenylpolysiloxane Silicone resin: 50% solution, in M3T, of a silicone network compound (trimethylsiloxysilicate) with a ratio, [Me₃SiO_{1/2}]/ [SiO₂], of 0.8 KF6012 (produced by Shin-Etsu Chemical Co., Ltd.): Polyoxyethylene/polyoxypropylene/methylpolysiloxane copolymer (HLB = 7.0) | |

### (Preparation Method)

A: Components 1 - 7 were mixed while heating and Component 8 was dispersed homogeneously.
B: Components 9 - 11 and Component 13 were mixed while heating.
C: While stirring, B was added portionwise to A to emulsify and, after cooling, Component 12 was added to obtained a sunscreen agent (milky lotion).

### Evaluation results of the above Exanples 11-23 and Comparative Examples 22-29.

| | No irritation to the skin | Comfortable to apply | Suitable as a cosmetic base | No straining after applied |
|---|---|---|---|---|
| UV-protective Cosmetic Base | | | | |
| Example 11 | 46 | 43 | 45 | 50 |
| Comp.Ex*.22 | 42 | 34 | 35 | 44 |
| Example 12 | 46 | 37 | 46 | 50 |
| Comp.Ex.23 | 45 | 35 | 40 | 36 |

| Whitening Cream for Daytime Use | | | | |
|---|---|---|---|---|
| Example 13 | 44 | 41 | 40 | 48 |
| Example 14 | 43 | 42 | 44 | 49 |
| Example 15 | 42 | 41 | 40 | 48 |
| Comp.Ex 24 | 10 | 19 | 10 | 40 |
| Comp.Ex 25 | 11 | 29 | 13 | 48 |
| Comp.Ex 26 | 10 | 10 | 10 | 10 |
| Comp.Ex 27 | 10 | 10 | 10 | 10 |

| Sun Screen Agent | | | | |
|---|---|---|---|---|
| Example 16 | 39 | 37 | 39 | 43 |
| Comp.Ex 28 | 18 | 20 | 12 | 26 |
| Example 17 | 43 | 33 | 37 | 46 |
| Comp.Ex 29 | 44 | 30 | 32 | 33 |
| Example 18 | 44 | 38 | 38 | 47 |
| Example 19 | 44 | 41 | 43 | 46 |
| Example 20 | 41 | 40 | 40 | 44 |
| Example 21 | 39 | 39 | 44 | 40 |
| Example 22 | 42 | 40 | 41 | 48 |
| Example 23 | 39 | 42 | 41 | 46 |

| | | | | |
|---|---|---|---|---|
| * Comparative Example | | | | |

The evalution results show that Examples of the present invention had excellent performance in all the evaluaion items. Examples were superior to Comparative Examples in not only the lack of dry feel but also the feel to the touch such as smoothness in applying cosmetic, and moisturizing feel.

Further, the present Examples had excellent UV-ray protective effect.

It was found from the results of Comparative Examples 26 and 27, that 1,1,1,5,5,5-hexamethyl-3-trimethylsiloxy -3-hydroxytrisiloxane causes sensory and safety problems.

The results of Comparative Example 28 shows that volatile linear silicone irritates the skin and gives uncomfortable feel to the skin.

### Example 24: Suntan Cream

| (Components) | (%) |
|---|---|
| 1. Tetraquistrimethylsiloxysilane | 12.0 |
| 2. Methyltrimethicone | 3.0 |
| 3. KF96A-100 | 5.0 |
| 4. KP-562 | 0.5 |
| 5. Branched silicone type polyglyceryl modified silicone (HLB =5) | 2.2 |
| 6. KF6026 | 6.0 |
| 7. Palmitic acid | 0.2 |
| 8. Dimethyloctyl paraaminobenzoic acid | 0.5 |
| 9. 4-t-butyl-4'-methoxy-dibenzoylmethane | 0.5 |
| 10. Kaoline | 0.5 |
| 11. Iron oxide red | 0.2 |
| 12. Iron oxide yellow | 0.3 |
| 13. Iron oxide black | 0.1 |
| 14. Titanium oxide coated mica | 1.0 |
| 15. Sodium L-glutamate | 3.0 |
| 16. 1,3-butylene glycol | 5.0 |
| 17. Dioctadecyldimethyl ammonium chloride | 0.1 |
| 18. Antioxidant | q.s. |
| 19. Antiseptic | q.s. |
| 20. Perfume | q.s. |
| 21. Purified water | Balance |

| | |
|---|---|
| KF96F-100 (produced by Shin-Etsu Chemical Co., Ltd.): Dimethylpolysiloxane with a viscosity of 100 mm²/s KP-562 (produced by Shin-Etsu Chemical Co., Ltd.): Behenyl-modified acrylic silicone graft copolymer KF6017 (produced by Shin-Etsu Chemical Co., Ltd.): Polyoxyethylene/methylpolysiloxane copolymer (HLB = 4.6) KF6026 (produced by Shin-Etsu Chemical Co., Ltd.): Polyoxyethylenemethylsiloxane/polyoxypropyleneoleylmethyl siloxane/dimethylsiloxane copolymer (HLB = 4.7) | |

### (Preparation Method)

A: Components 1 - 9 and Components 18- 19 were dissolved by heating.
B: After stirring Component 17 and a portion of Component 21 while heating, Components 10 - 14 were added to disperse.
C: Components 15 - 16 and the remaining portion of Component 21 were dissolved homogeneously and combined with B.
D. While stirring, C was added portionwise to A to emulsify and, after cooling, Component 20 was added to obtain a suntan cream.

The suntan cream thus obtained did not change with temperature or time, showing no phase separation or powder agglomeration. Further, it extended well on the skin and gave pleasant moisturizing feel.

### Example 25: Foundation

| (Components) | (%) |
|---|---|
| 1. Methyltrimethicone | 30.0 |
| 2. Tetraquistrimethylsiloxysilane | 15.0 |
| 3. KF96A-6 | 5.0 |
| 4. KF6017 | 1.5 |
| 5. KF6026 | 0.5 |
| 6. Montmorillonite modified by octadecyldimethylbenzylammonium | 4.0 |
| 7. Titanium dioxide treated for hydrophobicity* | 10.0 |
| 8. Talc treated for hydrophobicity* | 6.0 |
| 9. Mica treated for hydrophobicity* | 6.0 |
| 10. Iron oxide red* treated for hydrophobicity | 1.6 |
| 11. Iron oxide yellow* treated for hydrophobicity | 0.7 |
| 12. Iron oxide black* treated for hydrophobicity | 0.2 |
| 13. Dipropylene glycol | 5.0 |
| 14. Methyl paraoxybenzoate | 0.3 |
| 15. 2-amino-2-methyl-1,3-propanediol | 0.2 |
| 16. Hydrochloric acid | 0.1 |
| 17. Perfume | q.s. |
| 18. Water | Balance |

| | |
|---|---|
| KF96F-100 (produced by Shin-Etsu Chemical Co., Ltd.): Dimethylpolysiloxane with a viscosity of 100 mm²/s KF6017 (produced by Shin-Etsu Chemical Co., Ltd.): Polyoxyethylene/methylpolysiloxane copolymer (HLB = 4.6) KF6026 (produced by Shin-Etsu Chemical Co., Ltd.): Polyoxyethylenemethylsiloxane/polyoxypropyleneoleylmethyl siloxane/dimethylsiloxane copolymer (HLB = 4.7) * Treatment for hydrophobicity: after adding 2% of methylhydrogenpolysiloxane to the powder, a heat treatment was applied. | |

### (Preparation Method)

A: Components 1 - 6 were mixed while heating and Components 7 - 12 were added to obtain a homogeneous mixture.
B: Components 13 - 16 and Component 18 were dissolved by heating (pH of the aqueous system: 9.0).
C. While stirring, B was added portionwise to A to emulsify and, after cooling, Component 17 was added to obtain a foundation.

The foundation thus obtained did not change with temperature or time, showing no phase separation or powder agglomeration. Further, the applied foundation stayed long.

### Example 26: Foundation

| (Components) | (%) |
|---|---|
| 1. KF96A-6 | 5.0 |
| 2. Tetraquistrimethylsiloxysilane | 4.0 |
| 3. Methyltrimethicone | 11.0 |
| 4. Squalane | 4.0 |
| 5. Neopentyl glycol dioctanoate | 3.0 |
| 6. Myristic acid isostearic acid diglyceride | 2.0 |
| 7. α-monoisostearyl glycerylether | 1.0 |
| 8. KF6015 | 1.0 |
| 9. Aluminum distearate | 0.2 |
| 10. Titanium dioxide treated for hydrophobicity* | 5.0 |
| 11. Cerisite treated for hydrophobicity* | 2.0 |
| 12. Talc treated for hydrophobicity* | 3.0 |
| 13. Iron oxide red treated for hydrophobicity* | 0.4 |
| 14. Iron oxide yellow treated for hydrophobicity* | 0.7 |
| 15. Iron oxide black treated for hydrophobicity* | 0.1 |
| 16. Magnesium sulfate | 0.7 |
| 17. Glycerin | 3.0 |
| 18. Antiseptic | q.s. |
| 19. Perfume | q.s. |
| 20. Purified water | Balance |

| | |
|---|---|
| KF6017 (produced by Shin-Etsu Chemical Co., Ltd.): Polyoxyethylene/methylpolysiloxane copolymer (HLB = 4.5) * Hydrophobic powder: powder was treated with 2%, based on the powder, of stearic acid. | |

### (Preparation Method)

A: Components 1 - 9 were mixed while heating and Components 10 - 15 were added to obtain a homogeneous mixture.
B: Components 16 - 18 and Component 20 were dissolved by heating.
C. While stirring, B was added portionwise to A to emulsify and, after cooling, Component 19 was added to obtain a foundation.

The foundation thus obtained did not change with temperature or time, showing no phase separation or powder agglomeration. Further, the applied foundation stayed long.

### Example 27: Foundation

| (Components) | (%) |
|---|---|
| 1. Tetraquistrimethylsiloxysilane | 15.0 |
| 2. Methyltrimethicone | 3.0 |
| 3. KF56 | 5.0 |
| 4. Sorbitan monoisostearate | 0.5 |
| 5. Diglyceryl monoisostearate | 0.5 |
| 6. KF6012 | 1.0 |
| 7. Octyl paramethoxycinnamate | 3.0 |
| 8. Titanium oxide | 10.0 |
| 9. Iron oxide red | 0.13 |
| 10. Iron oxide yellow | 0.3 |
| 11. Iron oxide black | 0.07 |
| 12. Talc | 2.5 |
| 13. Sorbitol | 2.0 |
| 14. Magnesium sulfate | 0.1 |
| 15. Ethanol | 10.0 |
| 16. Antiseptic | q.s. |
| 17. Perfume | q.s. |
| 18. Purified water | Balance |

| | |
|---|---|
| KF56 (produced by Shin-Etsu Chemical Co., Ltd.): Methylphenylpolysiloxane KF6012 (produced by Shin-Etsu Chemical Co., Ltd.): Polyoxyethylene/polyoxypropylene/methylpolysiloxane copolymer (HLB = 7.0) | |

### (Preparation Method)

A: Components 8 - 12 were mixed homogeneously.
B: Components 1 - 7 and Component 16 were mixed while heating and A was added to obtain a homogeneous dispersion.
C. Components 13 - 14 and Component 18 were heated and added to B to emulsify and, after cooling, Components 15 and 17 were added to obtain a foundation.

The foundation thus obtained was in an excellent emulsified state. It was hardly affected by temperature; it was not changed with time; it was found to be very stable, showing no phase separation or agglomeration.

### Example 28: Foundation

| (Components) | (%) |
|---|---|
| 1. Tetraquistrimethylsiloxysilane | 15.0 |
| 2. KF96A-6 | 5.0 |
| 3. Liquid paraffin | 3.0 |
| 4. KF6015 | 3.0 |
| 5. Palmitic acid | 0.5 |
| 6. Aerosil RY200 | 5.0 |
| 7. Titanium dioxide | 6.0 |
| 8. Iron oxide red | 0.25 |
| 9. Iron oxide yellow | 0.6 |
| 10. Iron oxide black | 0.12 |
| 11. Cerisite | 8.03 |
| 12. Dipropylene glycol | 10.0 |
| 113. Magnesium sulfate | 2.0 |
| 14. Antiseptic | q.s. |
| 15. Antioxidant | q.s. |
| 16. Perfume | q.s. |
| 17. Purified water | Balance |

| | |
|---|---|
| KF96A-6 (produced by Shin-Etsu Chemical Co., Ltd.): Dimethylpolysiloxane with a viscosity of 6 mm²/s KF6015 (produced by Shin-Etsu Chemical Co., Ltd.): Polyoxyethylene/methylpolysiloxane copolymer (HLB = 4.5) Aerosil RY200 (produced by Nippon Aerosil Co., Ltd.): Hydrophobic silica | |

### (Preparation Method)

A: Components 7 - 12 were mixed homogeneously.
B: Components 1 - 6 and Component 16 were mixed and heated to 70°C under stirring, to which A was added to obtain a homogeneous dispersion.
C. Components 13 - 15 and 18 were heated to 70°C and added to B to emulsify and, after cooling, Component 17 was added to obtain a foundation.

The foundation thus obtained was in an excellent emulsified state. The applied foundation stayed long. The foundation was hardly affected by temperature; it was found to be very stable.

### Example 29: Foundation

| (Components) | (%) |
|---|---|
| 1. Tetraquistrimethylsiloxysilane | 9.0 |
| 2. Decamethylcyclopentasiloxane | 3.0 |
| 5. Methyltrimethicone | 3.0 |
| 6. KF96A-6 | 3.0 |
| 7. Octyl paramethoxycinnamate | 3.0 |
| 8. 12-hydroxysteatic acid | 1.0 |
| 9. FL-100 | 15.0 |
| 10. FPD-6131 | 5.0 |
| 11. KMP590 | 3.0 |
| 12. Fine particle titanium dioxide treated with a fluorine compound* | 8.0 |
| 13. mica titanium treated with a fluorine compound* | 1.0 |
| 14. Titanium dioxide treated with a fluorine compound* | 5.0 |
| 15. Iron oxide red treated with a fluorine compound* | 0.9 |
| 16. Iron oxide yellow treated with a fluorine compound* | 2.0 |
| 17. Iron oxide black treated with a fluorine compound* | 1.0 |
| 18. Ethanol | 15.0 |
| 19. Glycerin | 3.0 |
| 20. Magnesium sulfate | 1.0 |
| 21. Antiseptic | q.s. |
| 22. Perfume | q.s. |
| 23. Purified water | Balance |

| | |
|---|---|
| KF96A-6 (produced by Shin-Etsu Chemical Co., Ltd.): Dimethylpolysiloxane with a viscosity of 6 mm²/s FL-100 (produced by Shin-Etsu Chemical Co., Ltd.): Trifluoropropylmethylsilicone FPD6131 (produced by Shin-Etsu Chemical Co., Ltd.): Polyoxyethylene/trifluoropropyl/methylpolysiloxane copolymer (HLB = 5.4) KMP590 (produced by Shin-Etsu Chemical Co., Ltd.): Spherical silicone resin powder * Treatment with a fluorine compound: coated with 5% of perfluoroalkylethylphosphate diethanolamine salt | |

### (Preparation Method)

A: Components 10 - 16 were mixed homogeneously.
B: Components 1 - 9 were mixed while heating to 70°C and A was added to obtain a homogeneous dispersion.
C. Components 17 - 20 and Component 22 were heated to 40°C and added portionwise to B to emulsify. After cooling, Component 21 was added to obtain a liquid foundation.

The foundation thus obtained was found to be very stable, showing no change with temperature or time.

### Example 30: Foundation

| (Components) | (%) |
|---|---|
| 1. Tetraquistrimethylsiloxysilane | 27.0 |
| 2. KF56 | 3.0 |
| 3. Glyceryl triisooctanoate | 10.0 |
| 4. KF6017 | 1.0 |
| 5. KF6026 | 1.0 |
| 6. Polyglyceryl monoisostearate | 3.0 |
| 7. Powder mixture treated for hydrophobicity* | 18.0 |
| 8. Iron oxide red | 1.2 |
| 9. Iron oxide yellow | 2.6 |
| 10. Iron oxide black | 0.2 |
| 11. 1,3-butylene glycol | 7.0 |
| 12. Sodium chloride | 0.5 |
| 13. Antiseptic | q.s. |
| 14. Perfume | q.s. |
| 15. Purified water | Balance |

| | |
|---|---|
| *:Powder mixture treated for hydrophobicity a. Fine particle titanium dioxide 8.0 b. Fine particle zinc oxide 4.0 c. Talc 3.0 d. Mica 3.0 KF56 (produced by Shin-Etsu Chemical Co., Ltd.): Methylphenylpolysiloxane KF6017 (produced by Shin-Etsu Chemical Co., Ltd.): Polyoxyethylene/methylpolysiloxane copolymer (HLB = 4.6) KF6026 (produced by Shin-Etsu Chemical Co., Ltd.): Polyoxyethylenemethylsiloxane/polyoxypropyleneoleylmethyl siloxane/dimethylsiloxane copolymer (HLB = 4.7) | |

### (Preparation Method)

A: Components a - d were mixed.
B: Components 1 - 6 were mixed and dissolved by heating and Components 7 - 10 were homogeneously dispersed.
C. Components 11 - 13 and Component 15 were mixed and then added to B to emulsify.
D. After cooling C, Component 14 was added to obtain a foundation.

The foundation thus obtained stuck to the skin to give a glossy finish which stayed long. No change was found with temperature change or with time, showing superior stability.

### Example 31: Hair Cream

| (Components) | (%) |
|---|---|
| 1. Tetraquistrimethylsiloxysilane | 9.0 |
| 2. Methyltrimethicone | 1.0 |
| 3. KF56 | 4.0 |
| 4. Squalane | 5.0 |
| 5. Silicone resin | 1.0 |
| 6. Glyceryl dioleate | 2.0 |
| 7. KF6017 | 2.0 |
| 8. KF6026 | 4.0 |
| 9. Sodium sorbitol sulfate | 2.0 |
| 10. Sodium chondroitin sulfate | 1.0 |
| 11. Sodium hyaluronate | 0.5 |
| 12. Propylene glycol | 3.0 |
| 13. Antiseptic | 1.5 |
| 14. Vitamin E acetate | 0.1 |
| 15. Antioxidant | q.s. |
| 16. Perfume | q.s. |
| 17. Purified water | Balance |

| | |
|---|---|
| KF56 (produced by Shin-Etsu Chemical Co., Ltd.): Methylphenylpolysiloxane Silicone resin: 50% solution, in M3T, of a silicone network compound (trimethylsiloxysilicate) with a ratio, (Me₃SiO_{1/2}]/[SiO₂], of 0.8 KF6017 (produced by Shin-Etsu Chemical Co., Ltd.): Polyoxyethylene/methylpolysiloxane copolymer (HLB = 4.6) KF6026 (produced by Shin-Etsu Chemical Co., Ltd.): Polyoxyethylenemethylsiloxane/polyoxypropyleneoleylmethyl siloxane/dimethylsiloxane copolymer (HLB = 4.7) | |

### (Preparation Method)

A: Components 1 - 8 and Components 13 and 14 were mixed while heating.
B: Components 9 - 12 and Component 17 were mixed to be dissolved.
C: While stirring, B was added portionwise to A to emulsify and, after cooling, Component 16 was added to obtain a hair cream.

The haircream did not changed with temperature or time. The applied haircream stayed long.

### Example 32: Mascara

| (Components) | (%) |
|---|---|
| 1. KP545 | 20.0 |
| 2. Dextrin palmitate/ ethylhexanoate | 8.0 |
| 3. Polyethylene wax | 4.0 |
| 4. Beeswax | 7.0 |
| 5. Lecithin | 0.5 |
| 6. Methyltrimethicone | balance |
| 7. Tetraquistrimethylsiloxysilane | 5.0 |
| 7. Isododecane | 20.0 |
| 8. Iron oxide | 5.0 |
| 9. AerosilRY200 | 3.5 |
| 10. Talc | 10.0 |

| | |
|---|---|
| KP545 (produced by Shin-Etsu Chemical Co., Ltd.): Acryl silicone copolymer resin/decamethylcyclopentadiloxane 30% solution AerosilRY200: (Nippon Aerosil Co., Ltd.) Hydrophobic silica | |

### (Preparation Method)

A: Components 1 - 8 were mixed to be dissolved.
B: Components 9 - 11 were added to A and dispersed with a roller.

The mascara thus obtained did not change with temperature or time; it was found to be very stable. The applied mascara stayed long.

### Example 33: Skin Cream

| (Components) | (%) |
|---|---|
| 1. Tetraquistrimethylsiloxysilane | 20.0 |
| 2. Glyceryl trioctanoate | 10.0 |
| 3. KF6017 | 1.5 |
| 4. KG6026 | 4.0 |
| 5. Phenyldimethylstearyl ammonium chloride | 1.0 |
| 6. 1,3-butylene glycol | 10.0 |
| 7. Maltitol | 10.0 |
| 8. Saponite | 1.5 |
| 9. Antiseptic | q.s. |
| 10. Perfume | q.s. |
| 11. Purified water | Balance |

| | |
|---|---|
| KF6017 (produced by Shin-Etsu Chemical Co., Ltd.): Polyoxyethylene/methylpolysiloxane copolymer (HLB = 4.6) KF6026 (produced by Shin-Etsu Chemical Co., Ltd.): Polyoxyethylenemethylsiloxane/polyoxypropyleneoleylmethylsiloxane/dimethylsiloxane copolymer (HLB = 4.7) | |

### (Preparation Method)

A: Components 1 - 5 and 9 were mixed while heating.
B: Components 6 - 8 and Component 11 were dissolved by heating.
C: While stirring, B was added portionwise to A to emulsify and, after cooling, Component 10 was added to obtain a cream.

The cream thus obtained did not change with temperature or times; it was found to be very stable. The applied cream stayed long.

### Example 34: Skin Cream

A mixed solution of methyltrimethicone(M3T) and tetraquistrimethylsiloxysilane(M4Q) in a ratio of 1:1 was used.

| (Components) | (%) |
|---|---|
| 1. The mixed solution | 10.0 |
| 2. KF96A-6 | 5.0 |
| 3. Liquid paraffin | 5.0 |
| 4. Aqueous dispersion of silicone elastomer spherical powder | 2.0 |
| 5. KF6017 | 3.0 |
| 6. KF6026 | 5.0 |
| 7. Sodium citrate | 2.0 |
| 8. 1,3-butylene glycol | 5.0 |
| 9. Antiseptic | q.s. |
| 10. Perfume | q.s. |
| 11. Purified water | Balance |

| | |
|---|---|
| KF96A-6 (produced by Shin-Etsu Chemical Co., Ltd.): Dimethylpolysiloxane with a viscosity of 6 mm²/s KF6017 (produced by Shin-Etsu Chemical Co., Ltd.): Polyoxyethylene/methylpolysiloxane copolymer (HLB = 4.6) KF6026 (produced by Shin-Etsu Chemical Co., Ltd.): Polyoxyethylenemethylsiloxane/ polyoxypropyleneoleylmethylsiloxane/dimethylsiloxane copolymer (HLB = 4.7) | |

### (Preparation Method)

A: Components 1 - 5 were mixed while heating.
B: Components 6 - 9 and Component 11 were dissolved by heating.
C: While stirring, B was added portionwise to A to emulsify and, after cooling, Component 10 was added to obtain a cream.

The cream thus obtained gave appropriate moisturing feel and stayed long. No change was found with temperature change or with time, showing superior stability.

### Example 35: Skin Cream

| (Components) | (%) |
|---|---|
| 1. Tetraquistrimethylsiloxysilane | 20.0 |
| 2. Liquid paraffin | 5.0 |
| 3. KF615A | 1.0 |
| 9. Magensium L-ascorbate phosphate | 3.0 |
| 5. Dipropylene glycol | 5.0 |
| 6. Glycerin | 5.0 |
| 7. Antiseptic | q.s. |
| 8. Perfume | q.s. |
| 9. Purified water | Balance |

| | |
|---|---|
| KF615A (produced by Shin-Etsu Chemical Co., Ltd.): Polyoxyethylene/polyoxypropylene/methylpolysiloxane copolymer (HLB = 14.0) | |

### (Preparation Method)

A: Components 1 - 3 were mixed homogeneously.
B: Components 5 - 7 were heated to obtain a homogeneous mixture.
C: Components 4 and 9 were dissolved homogeneously.
D: While stirring, B was added portionwise to A, and C was further added to emulsify. Then, Component 8 was added to obtain a cream.

The cream thus obtained had affinity for the skin. It did not change with temprature or time; it was found to be very stable.

### Example 36: Skin Cream

A mixed solution of methyltrimethicone and tetraquistrimethylsiloxysilane in a ratio of 7:3 was used.

| (Components) | (%) |
|---|---|
| 1. The mixed slution | 20.0 |
| 2. (KF56 | 5.0 |
| 3. KF6012 | 1.5 |
| 4. Dextrin fatty acid ester | 1.0 |
| 5. Glycerin | 5.0 |
| 6. Sodium chloride | 1.0 |
| 7. Antiseptic | q.s. |
| 8. Perfume | q.s. |
| 9. Purified water | Balance |

| | |
|---|---|
| KF56 (produced by Shin-Etsu Chemical Co., Ltd.): Methylphenylpolysiloxane KF6012 (produced by Shin-Etsu Chemical Co., Ltd.): Polyoxyethylene/polyoxypropylene/methylpolysiloxane copolymer (HLB = 7.0) | |

### (Preparation Method)

A: Components 1 - 4 were mixed while heating.
B: Components 5 - 7 and Component 9 were dissolved by heating.
C: While stirring, B was added portionwise to A to emulsify and, after cooling, Component 8 was added to obtain a cream.

The cream thus obtained had good usability. It was resistant to water and sweat and the applied cream stayed long. No change was found with temperature change or with time, showing superior stability.

### Example 37: Skin Cream

| (Components) | (%) |
|---|---|
| 1. Tetraquistrimethylsiloxysilane | 18.0 |
| 2. KF96A-100 | 2.0 |
| 3. Polypropylene glycol (3) myristylether | 0.5 |
| 4. KF6017 | 1.4 |
| 5. KF6026 | 2.5 |
| 6. Fine particle titanium dioxide treated for hydrophobicity* | 1.0 |
| 8. Glycerin | 3.0 |
| 9. 70% sorbitol | 5.0 |
| 10. Citric acid | 25.0 |
| 11. Sodium chloride | 0.6 |
| 12. Antiseptic | q.s. |
| 13. Perfume | q.s. |
| 14. 32% aqueous ammonia | 4.5 |
| 15. Purified water | Balance |

| | |
|---|---|
| KF96A-100 (produced by Shin-Etsu Chemical Co., Ltd.): Dimethylpolysiloxane with a viscosity of 100 mm²/s KF6017 (produced by Shin-Etsu Chemical Co., Ltd.): Polyoxyethylene/methylpolysiloxane copolymer (HLB = 4.6) KF6026 (produced by Shin-Etsu Chemical Co., Ltd.): Polyoxyethylenemethylsiloxane/ polyoxypropyleneoleylmethylsiloxane/dimethylsiloxane copolymer (HLB = 4.7) * :Fine particle titanium dioxide treated with aluminum stearate | |

### (Preparation Method)

A: Components 1 - 5 and 12 were mixed, and then Component 6 was mixed by stirring.
B: Components 7 - 11 and Components 13 - 14 were dissolved homogeneously.
C: B was added portionwise to A to emulsify to obtain a cream.

The cream thus obtained extended well in spite of a relatively large content of citiric acid. No change was found with temperature change or with time, showing superior stability.

### Example 38: Skin Cream

| (Components) | (%) |
|---|---|
| 1. Tetraquistrimethylsiloxysilane | 15.0 |
| 2. Decamethylcyclopentasiloxane | 1.0 |
| 3. KF96A-6 | 4.0 |
| 4. KF6012 | 5.0 |
| 5. POE(5) octyldodecyl ether | 1.0 |
| 6. Polyoxyethylene sorbitan monostearate (20E.0.) | 0.5 |
| 7. SUNSPHERE SZ-5 | 4.0 |
| 8. Silicone-treated fine particle titanium dioxide | 5.0 |
| 9. Liquid paraffin | 2.0 |
| 10. Macademian nut oil | 1.0 |
| 11. Scuttellaria Root Extract* | 1.0 |
| 12. Gentiana Extract** | 0.5 |
| 13. Ethanol | 5.0 |
| 14. 1,3-butylene glycol | 2.0 |
| 15. Antiseptic | q.s. |
| 16. Perfume | q.s. |
| 17. Purified water | Balance |

| | |
|---|---|
| KF96A-6 (produced by Shin-Etsu Chemical Co., Ltd.): Dimethylpolysiloxane with a viscosity of 6 mm²/s KF6012 (produced by Shin-Etsu Chemical Co., Ltd.): Polyoxyethylene/polyoxypropylene/methylpolysiloxane copolymer (HLB = 7.0) SUNSPHERE SZ-5 (Produced by Asahi Glass Company) : Silica with a particle size ranging from 0.01 to 10µm, encapsulating 50% of anhydrous silicic acid-treated zinc oxide * Scuttellaria Root Extract: extracted with a 50% aqueous 1,3-butylene glycol solution ** Gentiana Extract: extracted with a 20% aqueous ethanol solution | |

### (Preparation Method)

A: Components 7- 10 were mixed to be dispersed homogeneously.
B: Components 1 - 6 were mixed and A was added thereto.
C: Components 11 - 15 and Component 17 were mixed, to which B was added to emulsify.
D: After cooling C, Component 16 was added to obtain a cream.

The cream thus obtained stuck well to the skin and gave a glossy finish which stayed long. No change was found with temperature change or with time, showing superior stability.

### Example 39: Handcream

| (Components) | (%) |
|---|---|
| 1. Tetraquistrimethylsiloxysilane | 1.0 |
| 2. Methyltrimethicone | 11.0 |
| 3. α-olefin oligomer | 10.0 |
| 4. Silicone resin | 5.0 |
| 5. KF6017 | 1.9 |
| 6. KF6026 | 4.0 |
| 7. Distearyldimethyl ammonium chloride | 0.8 |
| 8. Vitamin E acetate | 0.1 |
| 9. Polyethylene glycol 4000 | 1.0 |
| 10. Glycerin | 10.0 |
| 11. Smectite | 1.2 |
| 12. Antiseptic | q.s. |
| 13. Perfume | q.s. |
| 14. Purified water | Balance |

| | |
|---|---|
| Silicone resin: 70% solution, in M3T, of a silicone network compound (trimethylsiloxysilicate) with a ratio, [Me₃SiO_{1/2}]/[SiO₂], of 1.15 KF6017 (produced by Shin-Etsu Chemical Co., Ltd.): Polyoxyethylene/methylpolysiloxane copolymer (HLB = 4.6) KF6026 (produced by Shin-Etsu Chemical Co., Ltd.): Polyoxyethylenemethylsiloxane/polyoxypropyleneoleylmethyl siloxane/dimethylsiloxane copolymer (HLB = 4.7) | |

### (Preparation Method)

A: Components 1 - 7 and Component 11 were mixed while heating.
B: Components 8 - 10 and Component 13 were mixed under heating to be dissolved.
C: While stirring, B was added portionwise to A to emulsify and, after cooling, Component 12. was added to obtain a handcream.

The cream thus obtained did not change with temperature or time; it was found to be stable. The applied cream stayed long.

### Example 40: Handcream

| (Components) | (%) |
|---|---|
| 1. Tetraquistrimethylsiloxysilane | 30.0 |
| 2. Liquid paraffin | 10.0 |
| 3. Amino-modified silicone gum | 15.0 |
| 4. KF6017 | 4.0 |
| 5. Distearyldimethyl ammonium chloride | 0.8 |
| 6. Vitamin E acetate | 0.1 |
| 7. Polyethylene glycol 4000 | 1.0 |
| 8. Glycerin | 10.0 |
| 9. Smectite | 1.2 |
| 10. Antiseptic | q.s. |
| 12. Perfume | q.s. |
| 13. Purified water | Balance |

| | |
|---|---|
| Amino-modified silicone gum: Amine equivalence of 70000 g/mol KF6017 (produced by Shin-Etsu Chemical Co., Ltd.): Polyoxyethylene/methylpolysiloxane copolymer (HLB = 4.6) | |

### (Preparation Method)

A: Components 1 and 3 were heated and mixed to be dissolved, and then Components 2, 4 - 6 and 10 were added while heating.
B: Components 7 - 9 and Component 12 were mixed while heating.
C: B was added portionwise to A to emulsify and, after cooling, Component 11 was added to obtain a handcream.

The cream thus obtained effectively protected hands from kitchen work. It did not change with temperature; it was found to be very stable.

### Example 41: Handcream (O/W)

| (Components) | (%) |
|---|---|
| 1. KP545 | 5.0 |
| 2. Tetraquistrimethylsiloxysilane | 4.5 |
| 3. Methyltrimethicone | 0.5 |
| 4. KSG16 | 2.5 |
| 5. α-olefin oligomer | 5.0 |
| 6. Vaseline | 5.0 |
| 7. Glyceryl triisooctanoate | 3.0 |
| 8. KF6017 | 0.5 |
| 9. Polyoxyethylene sorbitan monooleate | 1.0 |
| 10. Sepigel 305 | 2.0 |
| 11. 1,3-butylene glycol | 5.0 |
| 12. Glycerin | 5.0 |
| 13. Antiseptic | q.s. |
| 14. Perfume | q.s. |
| 14. Purified water | Balance |

| | |
|---|---|
| KP545 (produced by Shin-Etsu Chemical Co., Ltd.): 30% solution of acrylic silicone copolymer resin/decamethylcyclopentasiloxane KSG16 (produced by Shin-Etsu Chemical Co., Ltd.): Crosslinked dimethylpolysiloxane/dimethylpolysiloxane KF6017 (produced by Shin-Etsu Chemical Co., Ltd.): Polyoxyethylene/methylpolysiloxane copolymer (HLB = 4.6) Sepigel 305: Light liquid paraffin (produced by SEPPIC Inc.) | |

### (Preparation Method)

A: Components 1 - 8 were mixed homogeneously.
B: Components 9 - 12 and Component 14 were mixed homogeneously.
C: B was added to A to emulsify and Component 13 was added to obtain an O/W handcream.

The handcream thus obtained stuck to the skin very well and stayed long. No change was found with temperature change or with time, showing superior stability.

### Example 42: Handcream (O/W)

| (Components) | (%) |
|---|---|
| 1. KP545 | 5.0 |
| 2. Tetraquistrimethylsiloxysilane | 4.0 |
| 3. Decamethylcyclopentasiloxane | 1.0 |
| 4. KP561 | 8.0 |
| 5. Cetanol | 1.0 |
| 6. Glyceryl triisostearate | 5.0 |
| 7. Stearic acid | 3.0 |
| 8. Glyceryl monostearate | 1.5 |
| 9. KF6015 | 0.7 |
| 10. Sorbitan sesquioleate | 0.5 |
| 11. Polyoxyethylene sorbitan monooleate | 1.0 |
| 12. Sodium hydroxide (1% aqueous solution) | 10.0 |
| 13.1,3-butylene glycol | 5.0 |
| 14. Antiseptic | q.s. |
| 15. Perfume | q.s. |
| 16. Purified water | Balance |

| | |
|---|---|
| KP545 (produced by Shin-Etsu Chemical Co., Ltd.): 30% solution of acrylic silicone copolymer resin/decamethylcyclopentasiloxane KF6015 (produced by Shin-Etsu Chemical Co., Ltd.): Polyoxyethylene/methylpolysiloxane copolymer (HLB = 4.5) KP561 (produced by Shin-Etsu Chemical Co., Ltd.): Acrylic silicone copolymer resin: stearyl-modified acrylate silicone | |

### (Preparation Method)

A: Components 1 - 10 were mixed and dissolved by heating.
B: Components 11 - 13 and Component 15 were mixed and heated.
C: B was added to A to emulsify and Component 14 was added to obtain an O/W handcream.

The handcream thus obtained stuck to the skin very well and stayed long. No change was found with temperature change or with time, showing superior stability.

### Example 43: Moisturizing Cream

| (Components) | (%) |
|---|---|
| 1. Tetraquistrimethylsiloxysilane | 5.0 |
| 2. Methyltrimethicone | 5.0 |
| 3. KF56 | 3.0 |
| 4. Liquid paraffin | 5.0 |
| 5. Pentaerythritol tetra-2-ethylhexanoate | 3.0 |
| 6. Cetyl 2-ethylhexanoate | 5.0 |
| 7. KF6017 | 1.0 |
| 8. KMP594 | 2.5 |
| 9. Aerosil R972 | 2.0 |
| 10. Zinc stearate | 2.0 |
| 11. Vitamin E acetate | 3.0 |
| 12. Polyoxyethylene glycol 400 | 1.0 |
| 13. Sodium lactate | 1.0 |
| 14. 1,3-butylene glycol | 5.0 |
| 15. Antiseptic | q.s. |
| 16. Perfume | q.s. |
| 17. Purified water | Balance |

| | |
|---|---|
| KF56 (produced by Shin-Etsu Chemical Co., Ltd.): Methylphenylpolysiloxane KF6017 (produced by Shin-Etsu Chemical Co., Ltd.): Polyoxyethylene/methylpolysiloxane copolymer (HLB = 4.6) KMP594 (produced by Shin-Etsu Chemical Co., Ltd.): Spherical silicone elastomer resin powder Aerosil R972 (produced by Nippon Aerosil Corp.): Hydrophobic silica | |

### (Preparation Method)

A: Components 1 - 7 and Components 10 - 11 were mixed homogeneously and Components 8 - 9 were dispersed homogeneously.
B: Components 12 - 15 and Component 17 were added to be dissolved.
C: B was added portionwise to A to emulsify and after cooling, Component 16 was added to obtain a moisturizing cream.

The moisturing cream thus obtained extended very well. It gave to the skin moisturing feel and was not tacky. It did not change with temperature or time; it was found to be excellent in stability, as well as usability.

### Example 44: Aftershave Cream

| (Components) | (%) |
|---|---|
| 1. Methyltrimethicone | 30.0 |
| 2. Tetraquistrimethylsiloxysilane | 5.0 |
| 3. KF6017 | 2.9 |
| 4. KF6026 | 5.0 |
| 5. Polyethylene glycol (molecular weight: 400) | 5.0 |
| 6. Sodium L-glutamate | 2.0 |
| 7. Arantoin | 0.1 |
| 8. Aloe extract | q.s. |
| 9. Ethanol | 3.0 |
| 10. Antiseptic | q.s. |
| 11. Antioxidant | q.s. |
| 12. Perfume | q.s. |
| 13. Purified water | Balance |

| | |
|---|---|
| KF6017 (produced by Shin-Etsu Chemical Co., Ltd.): Polyoxyethylene/methylpolysiloxane copolymer (HLB = 4.6) KF6026 (produced by Shin-Etsu Chemical Co., Ltd.): Polyoxyethylenemethylsiloxane/polyoxypropyleneoleylmethylsiloxane/dimethylsiloxane copolymer (HLB = 4.7) | |

### (Preparation Method)

A: Components 1 - 4 were mixed while heating.
B: Components 5 - 11 and 13 were mixed while heating.
C: B was added portionwise to A to emulsify and, after cooling, Component 12 was added to obtain an aftershave cream.

The aftershave cream thus obtained kept moisturing feel after application. It was very stable.

### Example 45: Eye Wrinkle Cream

| (Components) | (%) |
|---|---|
| 1. Tetraquistrimethylsiloxysilane . | 3.0 |
| 2. Methyltrimethicone | 17.0 |
| 3. KF7312J | 5.0 |
| 4. KF6017 | 2.0 |
| 5. KF6026 | 5.0 |
| 6. Sodium chondroitin sulfate | 2.0 |
| 7. Sodium lactate | 1.0 |
| 8. Glycerin | 50.0 |
| 9. Antiseptic | q.s. |
| 10. Antioxidant | q.s. |
| 11. Perfume | q.s. |
| 12. Purified water | Balance |

| | |
|---|---|
| KF7312J (produced by Shin-Etsu Chemical Co., Ltd.): Silicone resin: 50% solution, in decamethylcyclopentasiloxane, of a silicone network compound (trimethylsiloxysilicate) with a ratio, (Me₃SiO_{1/2}] / [SiO₂], of 0.8 KF6017 (produced by Shin-Etsu Chemical Co., Ltd.): Polyoxyethylene/methylpolysiloxane copolymer (HLB = 4.6) KF6026 (produced by Shin-Etsu Chemical Co., Ltd.): Polyoxyethylenemethylsiloxane/polyoxypropyleneoleylmethylsiloxane/dimethylsiloxane copolymer (HLB = 4.7) | |

### (Preparation Method)

A: Components 1 - 5 and Component 10 were mixed while heating.
B: Components 6 - 9 and Component 12 were dissolved by heating.
C: While stirring, B was added portionwise to A to emulsify and, after cooling, Component 11 was added to obtain an eye wrinkle cream.

The eye wrinkle cream formed a durable film and did not change with temperature or time; it was found to be very stable.

### Example 46: Eye Shadow

| (Components) | (%) |
|---|---|
| 1. Tetraquistrimethylsiloxysilane | 12.0 |
| 2. Methyltrimethicone | 3.0 |
| 2. KF96A-6 | 10.0 |
| 3. KF6012 | 2.0 |
| 4. PEG(10) laurylether | 0.5 |
| 5. Silicone-treated chromium oxide* | 6.2 |
| 6. Silicone-treated ultramarine blue* | 4.0 |
| 7. Silicone-treated titanium-coated mica* | 6.0 |
| 8. Sodium chloride | 2.0 |
| 9. Propylene glycol | 8.0 |
| 10. Antiseptic | q.s. |
| 11. Perfume | q.s. |
| 12. Purified water | Balance |

| | |
|---|---|
| KF96A-6 (produced by Shin-Etsu Chemical Co., Ltd.): Dimethylpolysiloxane with a viscosity of 6 mm²/s KF6012 (produced by Shin-Etsu Chemical Co., Ltd.): Polyoxyethylene/polyoxypropylene/methylpolysiloxane copolymer (HLB = 7.0) * Silicone treatment: 3%, based on the powder, of methylhydrogenpolysiloxane was added to the powder, followed by heat treatment. | |

### (Preparation Method)

A: Components 1 - 5 were mixed, to which Component 6 - 8 were added and dispersed homogeneously.
B: Components 9 - 11 and Component 13 were dissolved homogeneously.
C: While stirring, B was added portionwise to A to emulsify and Component 12 was added to obtain an eyeshadow.

The eye shadow thus obtained gave a glossy finish which stayed long. No change was found with temperature change or with time, showing superior stability.

### Example 47: Eyeliner

| (Components) | (%) |
|---|---|
| 1. Tetraquistrimethylsiloxysilane | 5.0 |
| 2. Methyltrimethicone | 17.0 |
| 2. KF96A-6 | 5.0 |
| 3. Jojoba oil | 2.0 |
| 4. KF6017 | 1.0 |
| 5. Silicone-treated iron oxide black (Note) | 20.0 |
| 6. Ethanol | 5.0 |
| 7. Antiseptic | q.s. |
| 8. Purified water | Balance |

| | |
|---|---|
| KF96A-6 (produced by Shin-Etsu Chemical Co., Ltd.): Dimethylpolysiloxane with a viscosity of 6 mm²/s KF6017 (produced by Shin-Etsu Chemical Co., Ltd.): Polyoxyethylene/methylpolysiloxane copolymer (HLB = 4.6) (Note) Silicone-treated Iron oxide black: 2% of methylhydrogenpolysiloxane was added to iron oxide black, followed by heat treatment. | |

### (Preparation Method)

A: Components 1 - 5 were mixed while heating, to which Component 6 was added to be dispersed homogeneously.
B: Components 7 - 9 were dissolved by heating.
C: While stirring, B was added portionwise to A to emulsify to obtain an eyeliner.

The eyeliner thus obtained gave a glossy finish which stayed long and was durable. No change was found with temperature change or with time, showing superior stability..

### Example 48: Eyeliner

| (Components) | (%) |
|---|---|
| 1. Tetraquistrimethylsiloxysilane | 32.0 |
| 2. KF96A-6 | 5.0 |
| 3. Silicone-treated iron oxide black | 20.0 |
| 4. Vitamin E acetate | 0.2 |
| 5. Jojoba oil | 2.0 |
| 6. Bentonite | 3.0 |
| 7. KF6012 | 2.0 |
| 8. Ethanol | 10.0 |
| 9. 1,3-butylenle glycol | 10.0 |
| 10. Antiseptic | q.s. |
| 11. Perfume | q.s. |
| 12. Purified water | Balance |

| | |
|---|---|
| KF96A-6 (produced by Shin-Etsu Chemical Co., Ltd.): Dimethylpolysiloxane with a viscosity of 6 mm²/s KF6012 (produced by Shin-Etsu Chemical Co., Ltd.): Polyoxyethylene/polyoxypropylene/methylpolysiloxane copolymer (HLB = 7.0) | |

### (Preparation Method)

A: Components 1, 2, and 4 - 7 were mixed, to which Component 3 was added to be dispersed homogeneously.
B: Components 8 - 10 and Component 12 were mixed.
C: B was added portionwise to A to emulsify and, after cooling, Component 11 was added to obtain an eyeliner.

The eyeliner thus obtained gave a glossy finish which stayed long and was durable. No change was found with temperature change or with time, showing superior stability.

### Example 49: Antiperspirant

| (Components) | (%) |
|---|---|
| 1. Methyltrimethicone | 26.0 |
| 2. Tetraquistrimethylsiloxysilane | 4.0 |
| 3. KF6026 | 1.0 |
| 4. Polyoxyethylenesorbitan monooleate (20 E.O.) | 0.5 |
| 5. Aluminum zirconium tetrachlorohydrate glycine salt | 20.0 |
| 6. Purified water | Balance |

| | |
|---|---|
| KF6026 (produced by Shin-Etsu Chemical Co., Ltd.): Polyoxyethylenemethylsiloxane/polyoxypropyleneoleylmethyl siloxane/dimethysiloxane copolymer (HLB = 4.7) | |

### (Preparation Method)

A: Components 1 and 2 were mixed.
B: Component 4 was dissolved in Component 5, and Component 3 was added.
C: While stirring, B was added portionwise to A to emulsify to obtain an antiperspirant.

The antiperspirant thus obtained was not tacky. It extended well on the skin and did not make the skin look white. It gave refreshing feel to users. No change was found with temperature change or with time, showing superior stability.

### Example 50: Antiperspirant

| (Components) | (%) |
|---|---|
| 1. KSG-21 | 20.0 |
| 2. KSG-15 | 20.0 |
| 3. Tetraquistrimethylsiloxysilane | 10.0 |
| 4. Methyltrimethicone | 20.0 |
| 5. Aluminum zirconium tetrachlorohydrate [Aluminum Zirconium Tetrachlorohydrex GLY] | 20.0 |
| 6. KF-96A-6 | 10.0 |

| | |
|---|---|
| KSG21 (produced by Shin-Etsu Chemical Co., Ltd.): Crosslinked polyether-modified methylpolysiloxane/dimethylpolysiloxane KSG15 (produced by Shin-Etsu Chemical Co., Ltd.): Crosslinked polyether-modified methylpolysiloxane/decamethylcyclopentasiloxane KF96A-6 (produced by Shin-Etsu Chemical Co., Ltd.): Dimethylpolysiloxane with a viscosity of 6 mm²/s | |

### (Preparation Method)

A: Components 1 - 4 and Component 6 were mixed homogeneously.
B: Component 5 was added to A and dispersed by mixing.

The antiperspirant thus obtained was non-tacky and showed superior stability with no change with temperature or time.

### Example 51: Transparent Gel Cosmetic

| (Components) | (%) |
|---|---|
| 1. Tetraquistrimethylsiloxysilane | 10.0 |
| 2. KF615A | 10.0 |
| 3. 1,3-butylene glycol | 10.0 |
| 4. Polyethylene glycol 400 | 9.0 |
| 5. 2-hydroxyoctanoic acid | 1.0 |
| 6. Sorbitol (70% aqueous solution) | 10.0 |
| 7. Citric acid | q.s. |
| 8. Sodium citrate | q.s. |
| 9. Antiseptic | q.s. |
| 10. Perfume | q.s. |
| 11. Purified water | Balance |

| | |
|---|---|
| KF615A (produced by Shin-Etsu Chemical Co., Ltd.): Polyoxyethylene/polyoxypropylene/methylpolysiloxane copolymer (HLB = 14.0) | |

### (Preparation Method)

A: Components 3 - 11 were dissolved homogeneously.
B: Components 1 and 2 were mixed to obtain a homogeneous mixture.
C: While stirring, A was added portionwise to B to emulsify to obtain a transparent gel cosmetic.

The transparent gel cosmetic thus obtained had affinity for the skin. It showed superior stability with no change with temperature or time.

### Example 41: Milky Lotion

| (Components) | (%) |
|---|---|
| 1. Tetraquistrimethylsiloxysilane | 13.0 |
| 2. KF96A-6 | 6.0 |
| 3. Squalan | 5.0 |
| 4. Neopentylglycol dioctanoate | 3.0 |
| 5. α-monooleylglyceryl ether | 1.0 |
| 6. KF6017 | 2.0 |
| 7. Aluminum distearate | 0.2 |
| 8. Magnesium sulfate | 0.7 |
| 9. Glycerin | 5.0 |
| 10. Antiseptic | q.s. |
| 10. Perfume | q.s. |
| 11. Purified water | Balance |

| | |
|---|---|
| KF96A-6 (produced by Shin-Etsu Chemical Co., Ltd.): Dimethylpolysiloxane with a viscosity of 6 mm²/s KF6017 (produced by Shin-Etsu Chemical Co., Ltd.): Polyoxyethylene/methylpolysiloxane copolymer (HLB = 4.6) | |

### (Preparation Method)

A: Components 1 - 7 were mixed while heating.
B: Components 8 - 10 and Component 12 were dissolved while heating.
C: While stirring, B was added portionwise to A and, after cooling, Component 11 was added to obtain a milky lotion.

The milky lotion thus obtained had a low viscosity and fine texture. It extended well and gave a moisturizing and soft feel. The applied lotion stayed long. It had superior stability with no change with temperature or time.

### Example 53: Milky Lotion

| (Components) | (%) |
|---|---|
| 1. Tetraquistrimethylsiloxysilane | 11.0 |
| 2. Methyltrimethicone | 4.0 |
| 3. KF96A-6 | 6.0 |
| 4. Squalan | 5.0 |
| 5. Neopentylglycol dioctanoate | 3.0 |
| 6. α-monooleylglyceryl ether | 1.0 |
| 7. KF6026 | 1.5 |
| 8. KF6017 | 1.0 |
| 9. Aluminum distearate | 0.2 |
| 10. Dextrin fatty acid ester | 1.0 |
| 11. Magnesium sulfate | 0.7 |
| 12. Glycerin | 5.0 |
| 13. Antiseptic | q.s. |
| 14. Perfume. | q.s. |
| 15. Purified water | Balance |

| | |
|---|---|
| KF96A-6 (produced by Shin-Etsu Chemical Co., Ltd.): Dimethylpolysiloxane with a viscosity of 6 mm²/s KF6026 (produced by Shin-Etsu Chemical Co., Ltd.): Polyoxyethylenemethylsiloxane/polyoxypropyleneoleylmethyl siloxane/dimethylsiloxane copolymer (HLB = 4.7) KF6017 (produced by Shin-Etsu Chemical Co., Ltd.): Polyoxyethylene/methylpolysiloxane copolymer (HLB = 4.6) | |

### (Preparation Method)

A: Components 1 - 10 were mixed while heating.
B: Components 11- 13 and Component 15 were dissolved while heating.
C: While stirring, B was added portionwise to A to emulsify and, after cooling, Component 13 was added to obtain a milky lotion.

The milky lotion thus obatined gave refreshing feel. No change with temperature or time was found, showing superior stability.

### Example 54: Milky Lotion

| (Components) | (%) |
|---|---|
| 1. Tetraquistrimethylsiloxysilane | 10.0 |
| 2. KF56 | 5.0 |
| 3. Squalane | 5.0 |
| 4. Pentaneerythritol tetra-2-ethylhexanoate | 5.0 |
| 5. KF6017 | 3.0 |
| 6. KMP594 | 2.0 |
| 7. Aerosil R972 | 0.5 |
| 8. Magnesium ascorbate phosphate | 1.0 |
| 9. Sodium chloride | 1.0 |
| 10. Polyethylene glycol 11000 | 1.0 |
| 11. Propylene glycol | 8.0 |
| 12. Antiseptic | q.s. |
| 13. Perfume | q.s. |
| 14. Purified water | Balance |

| | |
|---|---|
| KF56 (produced by Shin-Etsu Chemical Co., Ltd.): Methylphenylpolysiloxane KF6017 (produced by Shin-Etsu Chemical Co., Ltd.): Polyoxyethylene/methylpolysiloxane copolymer (HLB = 4.6) KMP594 (produced by Shin-Etsu Chemical Co., Ltd.): Spherical silicone elastomer resin powder Aerosil R972 (produced by Nippon Aerosil Co., Ltd.): Hydrophobic silica | |

### (Preparation Method)

A: Components 1 - 4 were mixed homogeneously, to which Components 5 and 6 were added to disperse homogeneously.
B: Components 7 - 9 were added to Component 13 to be dissolved. Components 10 and 11 were mixed with each other homogeneously and added to the solution.
C: B was added portionwise to A to emulsify and, after cooling, Component 12 was added to obtain a milky lotion.

The milky lotion thus obtained was non-tacky and gave soft feel when applied. No change with temperature or time was found, showing superior stability.

### Example 55: Beautifying Liquid

| (Components) | (%) |
|---|---|
| 1. Tetraquistrimethylsiloxysilane | 12.0 |
| 2. Glyceryl triisooctanoate | 10.0 |
| 3. KF6017 | 2.0 |
| 4. KSG21 | 0.2 |
| 5. Glycerin | 10.0 |
| '6. Magnesium ascorbate phosphate | 3.0 |
| 7. Sodium chloride | 2.0 |
| 8. Antiseptic | q.s. |
| 9. Perfume | q.s. |
| 10. Purified water | Balance |

| | |
|---|---|
| KF6017 (produced by Shin-Etsu Chemical Co., Ltd.): Polyoxyethylene/methylpolysiloxane copolymer (HLB = 4.6) KSG21 (produced by Shin-Etsu Chemical Co., Ltd.): Crosslinked polyether-modified methylpolysiloxane/dimethylpolysiloxane | |

### (Preparation Method)

A: Components 1 - 4 were mixed while heating.
B: Components 5 - 8 and Component 10 were heated to be dissolved homogeneously.
C: While stirring, B was added portionwise to A to emulsify and, after cooling, Component 9 was added to obtain a beautifying liquid.

The beautifying liquid thus obtained had affinity for the skin. No change with temperature or time was found, showing superior stability.

### Example 56: Deodorant

| (Components) | (%) |
|---|---|
| 1. Tetraquistrimethylsiloxysilane | 3.0 |
| 2. KF96A-6 | 4.0 |
| 3. KF615A | 1.0 |
| 4. Propylene glycol | 31.0 |
| 5. Triclosan | 0.1 |
| 6. Glycerin | 15.0 |
| 7. Antiseptic | q.s. |
| 8. Perfume | q.s. |
| 9. Purified water | Balance |

| | |
|---|---|
| KF96A-6 (produced by Shin-Etsu Chemical Co., Ltd.): Dimethylpolysiloxane with a viscosity of 6 mm²/s KF615A (produced by Shin-Etsu Chemical Co., Ltd.): Polyoxyethylene/polyoxypropylene/methylpolysiloxane copolymer (HLB = 14.0) | |

### (Preparation Method)

A: Components 1 - 3 were mixed.
B: Components 5 was dissolved in Component 4, and then Components 6 - 9 were mixed.
C: B was added to A to emulsify while vigorous stirring.
D: 65 parts of C and 35 parts of a propellant(mixture of n-butane, isobutene, and propane) were put in an aerosol can to obtain a deodorant.

The deodorant thus obtained was non-tacky and had superior stability.

### Example 57: Aerosol Composition (astringent-deodorant)

| (Components) | (%) |
|---|---|
| 1. Silicone-treated mica | 3.0 |
| 2. Chlorohydroxyaluminum | 2.0 |
| 3. Isopropylmethylphenol | 0.3 |
| 4. Sorbitan sesquioleate | 0.2 |
| 5. Isopropylmyristate | 5.0 |
| 6. Tetraquistrimethylsiloxysilane | 5.0 |
| 7. Methyltrimethicone | 1.0 |
| 8. Perfume | q.s. |
| 9. Propellant | Balance |

### (Preparation Method)

A: Components 1 - 8 were mixed.
B: After putting A in an aerosol can, Component 9 was filled.

The aerosol composition thus obtained had high deodorizing effect and was not tacky when applied. Furthermore, it was easy to use because of high re-dispersability.

### INDUSTRIAL APPLICABILITY

As described above, tetraquistrimethylsiloxysilane provides a cosmetic with an appropriate volatility. It is a good dispersion medium for pigments and makes a stable emulsion, so that it is suitable for cosmetics. Tetraquistrimethylsiloxysilane obtained by the invented method has a high purity which renders it suitable for cosmetics.

## Claims

1. A process for making a cosmetic comprising tetraquistrimethylsiloxysilane which comprises subjecting tetraalkoxysilane represented by the following formula (A) and hexamethyldisiloxane represented by the following formula (B),
Si(OR)₄ (A)
wherein R is a monovalent hydrocarbon group having 1 to 10 carbon atoms,
(CH₃)₃SiOSi(CH₃)₃ (B)
to a reaction according to a method comprising the steps of
(1) mixing 2 to 10 moles of hexamethyldisiloxane and 0.01 to 0.5 moles of an acid catalyst, per mole of tetraalkoxysilane, and adjusting the temperature to a temperature ranging from 0 °C to below 30 °C,
(2) adding 1 mole of tetraalkoxysilane to the system obtained in the step (1),
(3) adding 2.5 to 10.0 moles of water to the system obtained in the step (2) while keeping the temperature of from 0 °C to below 30 °C and subjecting to a reaction for 30 minutes to 5 hours, and
(4) subjecting to a reaction for 30 minutes to 5 hours at a temperature of from 30 °C to 100 °C.

2. The process according to claim 1, wherein the tetraalkoxysilane is at least one selected from the group consisting of tetramethoxysilane, tetraethoxysilane, and tetrapropoxysilane.

3. The process according to claim 1 or 2, wherein a lower monohydric alcohol having 1 to 6 carbon atoms is also mixed in an amount of from 0.5 to 10 moles per mole of the tetraalkoxysilane in the step (1).

4. The process according to claim 3, wherein the lower monohydric alcohol is at least one selected from the group consisting of methanol, ethanol, and isopropanol.

5. The process according to any one of claims 1 to 4, wherein the acid catalyst is at least one selected from the group consisting of sulfuric acid, methane sulfonic acid and trifluoromethane sulfonic acid.

6. The process according to any one of claims 1 to 5, wherein, in the cosmetic, the amount of 1,1,1,5,5,5-hexamethyl-3-trimethylsiloxy-3-hydroxytrisiloxane represented by the following formula (3)
[(CH₃)₃SiO]₃SiOH (3)
is at most 1 mass% based on the total mass of the cosmetic.

## Patentansprüche

1. Verfahren zur Herstellung eines Kosmetikums, umfassend Tetraquistrimethylsiloxysilan, welches das Unterziehen von Tetraalkoxysilan, dargestellt durch die folgende Formel (A) und Hexamethylsiloxan dargestellt durch die folgende Formel (B),
Si(OR)₄ (A)
wobei R eine einwertige Kohlenwasserstoffgruppe mit 1 bis 10 Kohlenstoffatomen ist,
(CH₃)₃SiOSi(CH₃)₃ (B)
einer Reaktion nach einer Methode umfasst, die die Schritte umfasst:
(1) Mischen von 2 bis 10 Mol von Hexamethyldisiloxan und 0,01 bis 0,5 Mol eines sauren Katalysators pro Mol Tetraalkoxysilan, und Anpassen der Temperatur an eine Temperatur im Bereich von 0°C bis unter 30°C,
(2) Hinzufügen von 1 Mol Tetraalkoxysilan zu dem im Schritt (1) erhaltenen System,
(3) Hinzufügen von 2,5 bis 10,0 Mol Wasser zu dem in Schritt (2) erhaltenen System, wobei die Temperatur von 0°C bis unter 30°C gehalten wird und Unterziehen einer Reaktion für 30 Minuten bis 5 Stunden, und
(4) Unterziehen einer Reaktion für 30 Minuten bis 5 Stunden bei einer Temperatur von 30°C bis 100°C.

2. Verfahren nach Anspruch 1, wobei das Tetraalkoxysilan zumindest eines ausgewählt aus der Gruppe bestehend aus Tetramethoxysilane, Tetraethoxysilan und Tetrapropoxysilan ist.

3. Verfahren nach Anspruch 1 oder 2, wobei ein niederer, monohydrischer Alkohol mit 1 bis 6 Kohlenstoffatomen gleichfalls in einer Menge von 0,5 bis 10 Mol pro Mol Tetraalkoxysilan in Schritt (1) gemischt wird.

4. Verfahren nach Anspruch 3, wobei der niedere, monohydrische Alkohol zumindest einer ausgewählt aus der Gruppe bestehend aus Methanol Ethanol, und Isopropanol ist.

5. Verfahren nach einem der Ansprüche 1 bis 4, wobei der saure Katalysator zumindest einer ausgewählt aus der Gruppe bestehend aus Schwefelsäure, Methansulfonsäure und Trifluormethansulfonsäure ist.

6. Verfahren nach einem der Ansprüche 1 bis 5, wobei die Menge an 1,1,1,5,5,5-hexamethyl-3-trimethylsiloxy-3-hydroxytrisiloxan dargestellt durch die folgende Formel (3)
[(CH₃)₃SiO]₃SiOH (3)
in dem Kosmetikum höchstens 1 Massenprozent bezogen auf die Gesamtmasse an Kosmetikum beträgt.

## Revendications

1. Procédé de fabrication d'un cosmétique comprenant du tétraquistriméthylsiloxysilane qui comprend la réaction de tétraalcoxysilane représenté par la formule (A) suivante et d'hexaméthylsiloxane représenté par la formule (B) suivante,
Si(OR)4 (A)
où R est un groupe hydrocarboné monovalent ayant de 1 à 10 atomes de carbone,
(CH₃)₃SiOSi(CH₃)₃ (B)
selon un procédé comprenant les étapes consistant
(1) à mélanger de 2 à 10 moles d'hexaméthyldisiloxane et de 0,01 à 0,5 mole d'un catalyseur acide, par mole de tétraalcoxysilane, et à ajuster la température dans un intervalle de température de 0°C à moins de 30°C,
(2) à ajouter 1 mole de tétraalcoxysilane au système obtenu dans l'étape (1),
(3) à ajouter de 2,5 à 10,0 moles d'eau au système obtenu dans l'étape (2) tout en maintenant la température à de 0°C à moins de 30°C et à réaliser une réaction pendant de 30 minutes à 5 heures, et
(4) à réaliser une réaction pendant de 30 minutes à 5 heures à une température de 30°C à 100°C.

2. Procédé selon la revendication 1, dans lequel le tétraalcoxysilane est au moins un choisi dans le groupe constitué du tétraméthoxysilane, du tétraéthoxysilane, et du tétrapropoxysilane.

3. Procédé selon la revendication 1 ou 2, dans lequel on mélange également un alcool mono-atomique inférieur ayant de 1 à 6 atomes de carbone dans une quantité de 0,5 à 10 moles par mole du tétraalcoxysilane dans l'étape (1).

4. Procédé selon la revendication 3, dans lequel l'alcool mono-atomique inférieur est au moins un choisi dans le groupe constitué du méthanol, de l'éthanol, et de l'isopropanol.

5. Procédé selon l'une quelconque des revendications 1 à 4, dans lequel le catalyseur acide est au moins un choisi dans le groupe constitué de l'acide sulfurique, de l'acide méthanesulfonique et de l'acide trifluorométhanesulfonique.

6. Procédé selon l'une quelconque des revendications 1 à 5, dans lequel, dans le cosmétique, la quantité de 1,1,1,5,5,5-hexaméthyl₋3-triméthylsiloxy-3-hydroxytrisiloxane représenté par la formule (3) suivante
[(CH₃)₃SiO]_{3S}iOH (3),
est d'au plus 1 % en masse rapporté à la masse totale du cosmétique.
